# EUROPÄISCHE PATENTSCHRIFT

(11) **EP 2 636 444 B1**
(45) Veröffentlichungstag und Bekanntmachung des Hinweises auf die Patenterteilung: **24.02.2016**
(21) Anmeldenummer: 12001623.3
(22) Anmeldetag: 08.03.2012
(51) Int. Cl.: B01F 7/00, B01F 15/00, C12M 1/107

(54) **Biogasanlagen-Fermenterbehälter mit einer Serviceeinrichtung sowie Serviceeinrichtung**
Biogas facility fermenter container with a service device and service device
Récipient de fermentation pour installation de biogaz doté d'un dispositif de service et dispositif de service

(43) Veröffentlichungstag der Anmeldung: 11.09.2013
(73) Patentinhaber: Niederbacher, Michael, 39031 Bruneck (IT)
(72) Erfinder: Niederbacher, Michael, 39031 Bruneck (IT)
(74) Vertreter: Liebl, Thomas

(56) Entgegenhaltungen:
- EP-A1- 2 213 720
- DE-C1- 19 621 914
- DE-U1-202009 010 167

## Beschreibung

Die Erfindung betrifft einen Biogasanlagen-Fermenterbehälter mit einer Serviceeinrichtung nach dem Oberbegriff des Anspruchs 1 sowie eine Serviceeinrichtung zur Verwendung in einem Biogasanlagen-Fermenterbehälter nach dem Oberbegriff des Anspruchs 18.

Um an einem durch eine Rühreinheit gebildeten Tauchgerät eines Biogasanlagen-Fermenterbehälters Wartungs- und Servicearbeiten durchführen zu können, ohne dass der zuständige Monteur ständig den im Fermenterbehälter-Innenraum gebildeten Fermentationsgasen ausgesetzt ist, ist es aus der DE 197 14 342 C1 bereits bekannt, einen Dom als gasdichte Abdeckung über einer deckenseitigen Rührwerköffnung anzubringen. Ein oberer Stützstangenbereich einer Stützstange als Führungsmast, entlang dem das Tauchgerät höhenverstellbar geführt ist, ragt hierbei mit der Betätigungseinrichtung über eine Dachwand des Domes hinaus. Die Rühreinheit selbst kann mittels einer Höhenverstelleinrichtung aus dem Fermenterbehälter-Innenraum in den Innenraum des Domes bewegt werden und ist dort durch wenigstens eine gasdicht verschließbare Domöffnung zugänglich. Damit sollen sowohl einfache als auch umfangreiche Reparatur- bzw. Wartungsarbeiten vom Monteur nicht mehr im Fermenterbehälter-Innenraum selbst durchgeführt werden, sondern bequemer und einfacher und damit auch preisgünstiger von außen durch die Domöffnung hindurch durchgeführt werden können. Da bei einem derartigen Aufbau aber nach wie vor Gas aus der Gasphase oberhalb des zu vergärenden flüssigen Substrates durch die Rührwerköffnung hindurch in den Innenraum des Doms gelangen kann, ist bzw. sind die Monteure nach wie vor den Fermentationsgasen ausgesetzt. Um dies möglichst zu vermeiden, schlägt die DE 197 14 342 C1 weiter vor, die Rührwerköffnung von der nicht verschlossenen Domöffnung her mit auf den öffnungsseitigen Auflagekanten aufliegenden Brettern abzudecken, die weiter mit einer Gummimatte abgedeckt sind, welche Gummimatte eine dem Stützstangenquerschnitt entsprechende Aussparung aufweist. Eine derartige Abdichtung der Rührwerköffnung ist unpraktikabel und sehr zeitaufwändig, da hier mit zusätzlichen Brettern und Gummimatten hantiert werden muss. Diese Maßnahme zur Abdichtung der Rührwerköffnung zur Gasphase des Fermenterbehälters hin wird daher im praktischen Einsatzfall regelmäßig nicht verwendet, so dass die Monteure in unerwünschter Weise nach wie vor dem Gas ausgesetzt sind.

Zur Höhenverstellung der Rühreinheit ist eine Seilzuganordnung vorgesehen, bei der ein Seil mit einem ersten Seilende an dem Tauchmotor festgelegt ist, wobei das Seil von dort aus in etwa senkrecht nach oben über eine erste Umlenkrolle an einem von dem Führungsmast wegragenden Tragarm gasdicht in das Innere des Führungsmastes geführt ist. Im Inneren des Führungsmastes ist das Seil dann um eine zweite Umlenkrolle nach oben geführt, wo das zweite Seilende am oberen Führungsmastende an einer Seilwinde festgelegt ist. Diese Seilwinde ist mit einer Handkurbel als Betätigungseinrichtung von außerhalb des Doms als Serviceeinrichtung betätigbar.

Weiter ist zur gasdichten Durchführung des oberen Führungsmastbereiches durch die obere Domwand eine siphonartige Dichtung vorgesehen. Für das Verdrehen des Führungsmastes ist an dem über die Dachwand des Domes hinausragenden, oberen Führungsmastende eine mit der Dachwand verbindbare Rastvorrichtung vorgesehen.

Ein derartiger Aufbau mit domartigem Serviceschacht als Serviceeinrichtung, bei dem eine siphonartige Flüssigkeitsdichtung vorgesehen ist und zudem ein Seil als Höhenverstelleinrichtung durch den Führungsmast hindurch in den Fermenterbehälter-Innenraum zum Tauchmotor geführt ist, ist zum einen relativ aufwändig und bauteilintensiv und zum anderen auch relativ störanfällig.

Ein vom Prinzip ähnlicher Aufbau, bei dem ein Tauchmotorrührgerät ebenfalls über eine Serviceöffnung aus dem Fermenterbehälter-Innenraum herausbewegt wird, ist weiter auch aus der EP 2 174 704 A1 bekannt. Dort ist im normalen Tauchgerätebetrieb eine als Deckenöffnung bezeichnete Serviceöffnung des Fermenterbehälters durch eine horizontale Wartungsplatte und ein angrenzendes, die Ebene der Wartungsplatte überragendes Mastgehäuse gasdicht abgedeckt. Im Mastgehäuse ist ein Mastoberteil eines vertikalen Führungsmastes einschließlich einer Höhenverstelleinrichtung mit einer Seilrolle, einem zugeordneten Zugseil und gegebenenfalls einer Getriebeeinheit dergestalt eingehaust, dass das Mastgehäuse Seitenwände aufweist, nach unten zum Fermenterbehälter offen ist und oben durch eine Gehäuseabdeckung verschlossen ist, die ein Stützlager für das obere Mastende des Führungsmastes enthält. Zumindest eine Seitenwand des Mastgehäuses ist in unmittelbarer Nähe des Mastoberteils angeordnet und als bedarfsweise öffenbare vertikale Wartungswand ausgeführt. Im unteren Bereich der öffenbaren vertikalen Wartungswand grenzt die öffenbare horizontale Wartungsplatte an, so dass für eine Wartungsbereitstellung des Tauchgerätes, die Wartungswand und die Wartungsplatte zu öffnen ist und das Tauchgerät mittels der Seilrolle und dem Zugseil nach oben über die Ebene der geöffneten Podestplatte aus dem Fermenterbehälter ausgehoben werden kann, so dass das Tauchgerät im Bereich vor der geöffneten Wartungswand frei zugänglich ist. Auch bei einem derartigen Aufbau einer Serviceeinrichtung besteht jedoch wiederum das Problem, dass bei geöffneter bzw. abgenommener Wartungswand und Wartungsplatte die Fermentationsgase über die Decken- bzw. Serviceöffnung ungehindert entweichen können und damit eine gesundheitliche Beeinträchtigung für den am herausgehobenen Tauchgerät arbeitenden Monteur darstellen. Zudem ist auch hier mit dem Mastgehäuse ein nach wie vor relativ großbauender bauteilintensiver Aufwand erforderlich, um eine Service- und Wartungseinrichtung zur Verfügung zu stellen. Ein ähnlicher Aufbau ist auch aus der DE 20 2009 010 167 U1 bekannt, bei dem die Seilrolle außerhalb des Mastgehäuses angeordnet ist und in der Abdeckplatte ein länglicher Durchführungsschlitz vorgesehen ist, durch den das Seil hindurchgeführt ist und der ein Verschwenken der am Schwenkmast angeordneten Seilrolle erlaubt. In Verbindung mit einem Foliendach ist ein Podestgestell offenbart, das die Serviceöffnung aufweist.

Weiter ist aus der DE 10 2009 031 177 A1 eine Serviceeinrichtung mit einer Serviceeinheit und einem Servicebereich bekannt, wobei die Serviceeinheit beispielsweise durch einen domartigen Serviceschacht, wie vorstehend beschrieben, gebildet sein kann. An dieser Serviceeinheit ist eine bewegliche Dichtungseinrichtung angeordnet, die zwischen einer Ruhestellung und einer Dichtungsstellung hin- und herverlagert werden kann, wobei die Dichtungseinrichtung geeignet sein soll, den Servicebereich von dem Gasbereich in einer Dichtungsstellung im Wesentlichen gasdicht zu trennen. Die Dichtungseinrichtung ist hier durch eine Dichtungswand aus einem flexiblen Material gebildet, beispielsweise faltenbalgartig oder teleskopartig verlagerbar ausgebildet, wobei weiter ein Führungsseil vorgesehen ist, mit welchem die Dichtungseinrichtung zwischen der Ruhestellung und der Dichtungsstellung hin- und herbewegbar ist. In der Dichtungsstellung soll sich die Dichtungseinrichtung von der Serviceeinrichtung ausgehend nach unten erstrecken und auf dem zu vergärenden flüssigen Substrat aufliegen bzw. mit dem Rand ein wenig in den Fermentationsbereich eintauchen. Ein derartiger beweglicher Gasvorhang ermöglicht zwar Service- bzw. Wartungsarbeiten am aus dem Fermenterbehälter-Innenraum herausgehobenen Tauchgerät, ist jedoch relativ aufwändig in der Bedienung, um Gasdichtheit herzustellen und darüber hinaus auch sehr fehleranfällig bzw. störanfällig, weil sich Feststoffe, zum Beispiel in Verbindung mit Schwimmschichten, sowie generell Ablagerungen, zum Beispiel durch Schwefel, an dem Dichtungsvorhang bzw. der damit verbundenen Hebemechanik festsetzen bzw. anlagern können und damit die ordnungsgemäße Funktion des Dichtungsvorhangs beeinträchtigen können. Des Weiteren besteht die Gefahr, dass die flexible Wand des Dichtungsvorhangs im abgesenkten Zustand und damit in ihrer Dichtungsstellung, insbesondere in Verbindung mit niedrigen Füllstandshöhen, keine ausreichende Länge aufweist, um in das flüssige, zu vergärende Substrat einzutauchen und daher das Gas in unerwünschter Weise über die Serviceöffnung entweichen kann. Wird zur Vermeidung eines solchen Falles eine ausreichende Länge des Dichtungswandmaterials vorgehalten, besteht wiederum in Verbindung mit hohen Flüssigkeits- bzw. Substratfüllständen die Gefahr, dass das Dichtungselement aufschwimmt und gegebenenfalls sogar eine Beeinträchtigung des in der Nähe desselben angeordneten Rührwerks zu befürchten ist. Ein weiterer Nachteil besteht darin, dass durch den Druckunterschied zwischen der Innenseite des flexiblen Gasvorhangs und dessen Außenbereich die Gefahr besteht, dass sich der Gasvorhang in den Servicebereich verbiegen kann. Aufgrund der Flexibilität der Wände der verlagerbaren Dichtungseinrichtung besteht zudem im Fermentationsbetrieb nach wie vor die Gefahr, dass das Dichtungselement keine funktionssicher abdichtende Einrichtung ausbildet und nach wie vor eine unerwünscht hohe Gasmenge über eine derartige Gasschleuse aus der Serviceöffnung austreten kann. Der das Tauchmotorrührgerät tragende Führungsmast ist auch hier wiederum in einer Deckenwand des domartigen Serviceschachtes verdrehbar gelagert und abgestützt. Weitere Ausführungen sind hierzu nicht gemacht.

Des Weiteren ist aus der DE 199 51 959 A1 ein Wartungsschacht für Rührwerke in Biogas-Fermenterbehältern bekannt, bei dem in Verbindung mit einer Betondecke eine Serviceöffnung vorgesehen ist, in die ein Wartungsschacht dergestalt eingesetzt ist, dass dieser von der Fermenterbehälter-Deckenwand nach unten dauerhaft in den Fermenterbehälter-Innenraum einragt. Die obere Seite dieses Wartungsschachtes ist aus einer Stahlplatte gefertigt, die auf einer Seite, am äußersten Rand, eine Flüssigkeitsdichtung für das Führungsrohr des Rührwerkes mit oberem Lager für das Führungsrohr sowie das Führungsrohr selbst einschließlich Seilwinde und Konsole mit Montageseilrolle aufnimmt. Die Konsole erlaubt das Drehen des Führungsrohres mit dem Rührwerk. Die Montageseilrolle soll das Anheben des Rührwerks über die Behälteroberfläche hinaus ermöglichen. Unmittelbar vor der Flüssigkeitsdichtung befindet sich ein eine Serviceöffnung verschließender Deckel, der im geöffneten Zustand die Durchführung von Wartungs- und Montagearbeiten erlaubt. Der Wartungsschacht selbst kann starr ausgebildet sein oder aber auch mit einem vertikal beweglichen Unterteil versehen sein. Mit einem derartigen Wartungsschacht kann das Tauchmotorrührgerät ohne großen Gasverlust ausgehoben werden, da der Wartungsschacht den restlichen Gasphasenbereich des Fermenterbehälter-Innenraums zuverlässig von dem wartungsschachtseitigen Gasphasenbereich abtrennt. Die Flüssigkeitsdichtung, die Bestandteil der Höhenverstelleinrichtung für das Tauchmotorrührgerät bildende Seilwinde und der Führungsmast selbst sind im Wesentlichen analog der eingangs beschriebenen DE 197 14 342 C1 ausgebildet.

Weiter ist aus der EP 2 213 720 A1 eine Biogasanlage mit einem in einem Fermenterbehälter an einem Führungsmast mit einem Schiebeteil höhenverstellbar geführten Tauch-Rüh rwerk und einer zugeordneten Stelleinrichtung bekannt. Die Stelleinrichtung weist eine sich parallel zum Führungsmast und wenigstens über den Stellweg erstreckende Spindel sowie weiter am Schiebeteil eine Spindelmutter auf. Durch eine motorische oder handbetätigte Relativbewegung zwischen der Spindel und der Spindelmutter wird das Schiebeteil zusammen mit dem daran angeordneten Tauch-Rührwerk entlang der Spindel verfahren. Ausgehend von einer Oberkante einer Behälterseitenwand ist ein in den Behälterbereich ragendes, horizontales Podest angebracht, an dem randseitig ein Foliendach angeschlossen ist, welches den Behälter abdeckt. Über eine Podestöffnung als Serviceöffnung ist ein Mastgehäuse im Sinne der EP 2 174 704 A1 angebracht, welches zudem durch ein gasdichtes Haubenteil über der Podestöffnung abgedeckt ist. Der Führungsmast mit rechteckigem oder quadratischem Durchmesser ist an einem Fußpunkt am Behälterboden und einem Kopfteil am Dachwandbereich des Mastgehäuses um seine Vertikalachse schwenkbar gelagert. Als motorischer Schwenkantrieb ist hier ein Hydraulikmotor am Mastgehäuse sowie ein Kettentrieb eingesetzt. Alternativ könnte beispielsweise auch ein Elektromotor mit einem Schneckentrieb verwendet werden. Die Höhenverstelleinrichtung für das Tauchrührwerk umfasst einen durch einen Hydraulikmotor gebildeten motorischen Spindeldrehantrieb, der am Führungsmast angeordnet ist. Von oben, außerhalb des Gasbereichs sind gasdichte Energieleitungen als Hydraulikschläuche jeweils zum hydraulischen Rührwerkantrieb, dem hydraulischen Spindelantriebsmotor und dem hydraulischen Schwenkantrieb für den Führungsmast geführt.

Demgegenüber ist es Aufgabe der vorliegenden Erfindung, einen Biogasanlagen-Fermenterbehälter mit einer Serviceeinrichtung sowie eine Serviceeinrichtung zur Verwendung in einem Biogasanlagen-Fermenterbehälter vorzuschlagen, mittels dem das Verschwenken eines Führungsmastes und die Höhenverstellung eines Tauchgerätes auf bauteiltechnisch einfache und kompakte Weise durchgeführt werden kann, und zwar insbesondere auch in Verbindung mit einer Serviceeinrichtung, mittels der Wartungs- und Servicearbeiten an einem Tauchgerät, auf einfache und zuverlässige Weise ohne nennenswerte Beeinträchtigung durch aus dem Fermenterbehälter entweichendes Gas durchgeführt werden kann.

Diese Aufgabe wird bezüglich des Biogasanlagen-Fermenterbehälters gelöst mit den Merkmalen des Patentanspruchs 1. Bezüglich der Serviceeinrichtung wird diese Aufgabe gelöst mit den Merkmalen des Patentanspruchs 18. Bevorzugte Ausgestaltungen sind jeweils Gegenstand der Unteransprüche.

Gemäß Patentanspruch 1 ist ein Biogasanlagen-Fermenterbehälter mit einer Serviceeinrichtung vorgesehen, die eine mittels einer Abdeckeinrichtung verschließbare, vorzugsweise gasdicht verschließbare, deckenseitige Serviceöffnung aufweist, durch die ein an einem Führungsmast höhenverstellbar geführtes Tauchgerät, insbesondere ein Tauchrührgerät oder eine Tauchpumpe, für Wartungs- und Servicearbeiten zugänglich ist, insbesondere im Wesentlichen gasdicht aus dem Fermenterbehälter heraus und wieder in den Fermenterbehälter hinein bewegbar ist. Der Führungsmast ist mittels einer Lagerwelle verdrehbar und/oder verschwenkbar in einer Lagerstelle der Serviceeinrichtung gelagert, wobei die Lagerwelle zum Verdrehen oder Verschwenken des Führungsmastes mittels einer außerhalb des Fermenterbehälters angeordneten Führungsmast-Schwenkeinrichtung gekoppelt ist. Ferner ist auch das Tauchgerät mit einer außerhalb des Fermenterbehälters angeordneten Tauchgerät-Höhenverstelleinrichtung gekoppelt, die eine Antriebswelle aufweist, die zusammen mit der Lagerwelle der Führungsmast-Schwenkeinrichtung in einer Hohlwellenanordnung durch die Lagerstelle der Serviceeinrichtung hindurch in den Fermenterbehälter-Innenraum geführt ist. Die Antriebswelle wirkt zudem im Fermenterbehälter-Innenraum mittelbar oder unmittelbar mit einem Verstellelement zusammen, mittels dem das Tauchgerät am Führungsmast höhenverstellbar ist.

Mit einer derartigen Hohlwellenanordnung wird eine einfach abzudichtende, baulich kompakte und mit geringem Aufwand herstellbare einzige Lagerstelle an einer Serviceeinrichtung für einen Biogasanlagen-Fermenterbehälter zur Verfügung gestellt, die zudem flexibel für unterschiedlichste Einsatzfälle einsetzbar sind. So zum Beispiel insbesondere in Verbindung mit einer durch eine im Wesentlichen ebene, großflächige und vorzugsweise begehbare Montage- und Podestplatte als Serviceeinrichtung bzw. Bestandteil der Serviceeinrichtung, in die eine mittels eines bevorzugt flachen, plattenartigen Deckels abdeckbare Serviceöffnung integriert ist, so dass auch ein für besonders beengte Einbauverhältnisse geeigneter und/oder wenig hochbauender Aufbau einer Serviceeinrichtung geschaffen wird, der sich aufgrund seiner Bauteil- und Funktionsintegration und seiner kompakten Bauweise zudem auch durch geringen Bauteil- und Montageaufwand und damit insgesamt geringe Kosten auszeichnet.

Insbesondere kann eine derartige Serviceeinrichtung zudem auch sehr gut mit einer nachfolgend noch näher erläuterten Gasschürzenanordnung kombiniert werden, so dass bei einem derartigen Aufbau auch Wartungs- und Servicearbeiten ohne aufwändigen Serviceschacht funktionssicher mit geringer bzw. kaum Gasbelastung für einen Monteur durchgeführt werden können.

Gemäß einer besonders bevorzugten konkreten Ausgestaltung wird vorgeschlagen, dass das Verstellelement durch eine sich parallel zum Führungsmast erstreckende und dort drehbar gelagerte Gewindespindel gebildet ist, wobei die Gewindespindel eine tauchgeräteseitige Spindelmuttereinrichtung so durchgreift, dass das Tauchgerät bei einer Drehbetätigung der Gewindespindel mittels der Antriebswelle, in Abhängigkeit von der Drehrichtung der Gewindespindel, entlang des Führungsmastes nach oben bzw. nach unten verlagerbar ist. Mit einer derartigen Gewindespindel ist das Tauchgerät in einer bestimmten gewählten Höhenposition durch die Fixierung und Selbsthemmung der Gewindeverbindung sowohl nach unten als auch nach oben zuverlässig festgelegt und abgestützt, wodurch ein stabiler Betrieb des Tauchgerätes in allen Höhenpositionen mit reduzierter Neigung zu unerwünschten und materialbelasteten Eigenschwingungen möglich ist.

Gemäß einer besonders bevorzugten konkreten Ausgestaltung wird vorgeschlagen, dass die Hohlwellenanordnung so ausgebildet ist, dass entweder die Lagerwelle der Führungsmast-Schwenkeinrichtung oder die Antriebswelle der Tauchgerät-Höhenverstelleinrichtung als Hohlwelle ausgebildet ist, die in der Lagerstelle der Serviceeinrichtung verdrehbar gelagert ist und in der die jeweils andere Welle verdrehbar aufgenommen und gelagert ist. Die verdrehbare Lagerung bzw. Aufnahme kann dabei zum Beispiel über besonders gut abdichtbare Gleit- oder Kugellager erfolgen. Eine derartige konkrete Hohlwellenanordnung zeichnet sich durch eine besonders kompakte Bauweise aus, wobei je nach konkreter Ausgestaltung entweder die Lagerwelle oder die Antriebswelle als Hohlwelle ausgebildet sein kann. Eine derartige Hohlwellenanordnung lässt sich zudem sehr gut abdichten.

Um das Verstellelement bzw. in der zuvor geschilderten konkreten bevorzugten Ausgestaltung die Gewindespindel anzutreiben, kann die Antriebswelle der Tauchgerät-Höhenverstelleinrichtung im Fermenterbehälter-Innenraum ein Antriebswellen-Antriebselement, zum Beispiel ein Antriebszahnrad oder dergleichen, aufweisen, das mittelbar oder unmittelbar mit einem mit dem Verstellelement mittelbar oder unmittelbar gekoppelten Verstellelement-Antriebselement zusammenwirkt. Bei dem Verstellelement kann es sich bevorzugt um eine Gewindespindel handeln, die dann mit einem Gewindespindel-Antriebselement, zum Beispiel einem Gewindespindel-Zahnrad, gekoppelt ist. Ein derartiges zum Beispiel Gewindespindel-Zahnrad kann gemäß einer besonders bevorzugten Ausgestaltung direkt an der Gewindespindel angeordnet sein.

Wie zuvor ausgeführt, können die Antriebselemente durch zum Beispiel Antriebszahnräder oder dergleichen gebildet sein, die Bestandteil einer Getriebeanordnung sein können. In einer äquivalenten und analogen Weise können derartige durch Zahnräder gebildete Antriebseinheiten aber auch durch Umschlingungstriebe, wie beispielsweise Kettentriebe oder Riementriebe oder dergleichen gebildet sein.

Gemäß einer besonders bevorzugten konkreten Ausgestaltung wird vorgeschlagen, dass die Lagerwelle der Führungsmast-Schwenkeinrichtung als Hohlwelle ausgebildet ist, durch die die Antriebswelle der Tauchgerät-Höhenverstelleinrichtung in dem Fermenterbehälter-Innenraum geführt ist, wobei die Lagerwelle dann mit einer mit dem Führungsmast unmittelbar oder mittelbar verbundenen Lagerbrücke gekoppelt ist, die das Antriebswellen-Antriebselement, zum Beispiel ein Antriebszahnrad, berührungsfrei überbrückt bzw. in einem brückenseitigen Frei- oder Aufnahmeraum aufnimmt. Dabei ist auf einfache Weise sichergestellt, dass, bei in etwa mittig und zentral durch die Hohlwellenanordnung durchgeführtem bzw. angeordnetem Antriebselement, das regelmäßig seitlich versetzt zum Führungsmast angeordnete Verstellelement, zum Beispiel eine Gewindespindel, funktionssicher und kollisionsfrei mit dem Schwenkantrieb für den Führungsmast ausgebildet sein kann.

In einer alternativen konkreten Ausgestaltung ist dagegen vorgesehen, dass die Antriebswelle der Tauchgerät-Höhenverstelleinrichtung als das Antriebswellen-Antriebselement aufweisende Hohlwelle ausgebildet ist, durch die die Lagerwelle der Führungsmastschwenkeinrichtung in dem Fermenterbehälter-Innenraum geführt ist. Ein derartiger Aufbau ist besonders einfach und kompakt herzustellen, da hier die Lagerwelle dann unmittelbar und direkt zu dem Führungsmast geführt werden kann.

Die Führungsmast-Schwenkeinrichtung kann für einen manuellen Betrieb beispielsweise durch einen mit der Lagerwelle gekoppelten handbetätigbaren Schwenkhebel gebildet sein. Alternativ dazu kann die Führungsmast-Schwenkeinrichtung aber auch durch einen Antriebsmotor gebildet sein, wobei in diesem Fall dann eine Abtriebswelle des Antriebsmotors die Lagerwelle der Führungsmast-Schwenkeinrichtung ausbildet oder Bestandteil der Lagerwelle der Führungsmast-Schwenkeinrichtung ist oder mit der Lagerwelle der Führungsmast-Schwenkeinrichtung unmittelbar oder mittelbar drehübertragen gekoppelt ist.

In analoger Weise dazu kann auch die Tauchgerät-Höhenverstelleinrichtung durch eine mit der Antriebswelle gekoppelte handbetätigbare Kurbeleinrichtung gebildet sein. Alternativ dazu kann die Tauchgerät-Höhenverstelleinrichtung aber auch durch einen Antriebsmotor gebildet sein, wobei in diesem Fall dann eine Abtriebswelle des Antriebsmotors die Antriebswelle der Tauchgerät-Höhenverstelleinrichtung ausbildet oder Bestandteil der Antriebswelle der Tauchgerät-Höhenverstelleinrichtung ist oder mit der Antriebswelle der Tauchgerät-Höhenverstelleinrichtung unmittelbar oder mittelbar bewegungsübertragend, insbesondere drehübertragend, gekoppelt ist.

Wie die soeben gemachten Ausführungen zeigen, kann somit sowohl die Führungsmast-Schwenkeinrichtung als auch die Tauchgerät-Höhenverstelleinrichtung in Verbindung mit einer erfindungsgemäßen einzigen Lagerstelle auf einfache Weise entweder handbetätigbar oder motorisch betätigbar ausgebildet sein. Auch Mischformen sind denkbar, das heißt, dass zum Beispiel die Führungsmast-Schwenkeinrichtung handbetätigbar ausgebildet sein kann, während beispielsweise die Tauchgerät-Höhenverstelleinrichtung motorisch angetrieben ausgebildet sein kann. Selbstverständlich ist auch eine entsprechend umgekehrte Ausgestaltung möglich, bei der die Tauchgerät-Höhenverstelleinrichtung handbetätigbar ausgebildet ist und die Führungsmast-Schwenkeinrichtung motorisch angetrieben ausgebildet ist. Mit der erfindungsgemäßen Lösung ergeben sich somit erhöhte Flexibilitäten und konstruktive Freiheiten bei der konkreten Ausführung und Auslegung einer Serviceeinrichtung an einem Biogasanlagen-Fermenterbehälter.

Insbesondere in Verbindung mit einer automatisierten motorischen Betätigung der Führungsmast-Schwenkeinrichtung und/oder der Tauchgerät-Höhenverstelleinrichtung ergibt sich die Möglichkeit, diese über ein Steuer- und/oder Regelgerät automatisiert zu betätigen, zum Beispiel von einer Wartungs- und Kontrollstation aus, so dass nicht unbedingt ein Arbeiten vor Ort erforderlich ist.

Insbesondere um die Hohlwellenanordnung gegenüber äußere, witterungsseitige Einflüsse abzuschirmen, kann gemäß einer weiteren bevorzugten Ausgestaltung zudem vorgesehen sein, dass die Hohlwellenanordnung so ausgebildet ist, dass die Hohlwelle in einer der Lagerstelle der Serviceeinrichtung zugeordneten Hülse (Gehäusehülse) drehbar bzw. verschwenkbar gelagert ist. Diese Lagerung kann zum Beispiel wiederum durch eine besonders einfach abdichtbare Gleitlagerung oder Kugellagerung ausgebildet sein. Diese Hülse weist ferner einen Befestigungsflansch auf, der integral mit dieser verbunden sein kann oder aber auch durch ein separates Bauteil gebildet sein kann. Mittels diesem Befestigungsflansch kann dann die Hülse um die Lagerstelle der Serviceeinrichtung herum, insbesondere um die Lagerstelle einer die Serviceeinrichtung aufweisenden Podestplatte herum, wie diese nachfolgend noch näher beschrieben wird, fest und unverdrehbar mit der Serviceeinrichtung bzw. an der Serviceeinrichtung verbunden werden. In Verbindung mit dieser Hülse ist zudem noch eine weitere Ausgestaltung besonders vorteilhaft, bei der zum Beispiel im Falle von Antriebsmotoren vorgesehen sein kann, dass diese über ein Gestell oder eine Stütze an der Hülse abgestützt oder festgelegt werden. Die entsprechenden Getriebe bzw. Zahnräder oder Abtriebs- und Antriebselemente können dabei in einem separaten, ebenfalls mit der Hülse oder mit dem verbindbaren Gehäuse angeordnet werden.

Die Antriebsmotoren selbst können grundsätzlich auf unterschiedliche Weise ausgebildet sein, zum Beispiel als mittels eines Arbeitsmediums hydraulisch oder pneumatisch betreibbare Antriebsmotoren. Besonders bevorzugt ist jedoch eine Ausgestaltung, bei der die Antriebsmotoren durch einfach und funktionssicher bedienbare Elektromotoren gebildet sind.

Wie bereits zuvor angedeutet, ist es besonders vorteilhaft, wenn um die Serviceöffnung der Serviceeinrichtung herum ein in der Dichtstellung schachtartig nach unten in den Fermenterbehälter-Innenraum einragende Gasschürze angeordnet ist, die wenigstens bei Wartungs- und Servicearbeiten im das im Fermenterbehälter aufgenommene, zu vergärende flüssige Substrat eintaucht und den von der Gasschürze ringförmig umschlossenen Gasphasenbereich unterhalb der öffenbaren Serviceöffnung sowie oberhalb des Substrates gasdicht von dem restlichen Gasphasenbereich des Fermenterbehälter-Innenraum abtrennt. Besonders bevorzugt ist dabei vorgesehen, dass diese Gasschürze durch einen starren, nicht verlagerbaren und unflexiblen sowie von der Serviceöffnung dauerhaft in Richtung Fermenterbehälter-Innenraum abragenden rohrstutzenartigen Kragen gebildet ist. Ein solcher starrer Kragen kann zum Beispiel aus einem stabilen Material, zum Beispiel einem Metall, höchst bevorzugt aus einem korrosionsbeständigen Edelstahlmaterial, hergestellt sein und bringt den Vorteil mit sich, dass mit einem derartigen starren, nicht verlagerbaren Kragen als Gasschürze auf einfache Weise sichergestellt werden kann, dass durch die fehlende Verlagerungsmöglichkeit der Gasschürze Störfälle bei der Abdichtung durch eine fehlerhafte oder gestörte Dichtelementverlagerung bereits von vorne herein ausgeschlossen sind. Zudem ist durch die starre, stabile Ausbildung des rohrstutzenartigen Kragens sichergestellt, dass dieser nicht überdrückt bzw. weggedrückt werden kann und dadurch gegebenenfalls eine Gefahr für einen Rührbetrieb darstellt.

Um auf einfache Weise sicherzustellen, dass mittels dem starren, rohrstutzenartigen Kragen eine zuverlässige Abdichtung des Servicebereiches erzielt wird, ist gemäß einer besonders bevorzugten Ausgestaltung der erfindungsgemäßen Lösung vorgesehen, dass der Serviceeinrichtung weiter eine Füllstands-Messeinrichtung zugeordnet ist oder eine solche aufweist, mittels der wenigstens bei Wartungs- und Servicearbeiten der Füllstand des Substrates im Fermenterbehälter erfasst und ein entsprechendes Füllstands-Messsignal als Höhen-Istwertsignal erzeugt wird, wobei weiter eine Steuereinrichtung vorgesehen ist, der das Füllstands-Messsignal zugeführt wird und die bei einem erfassten geringen Füllstand, bei dem die Gasschürze nicht in das Substrat eintaucht, wenigstens eine Förder- und/oder Pumpeinrichtung der Biogasanlage dergestalt ansteuert, dass der Füllstand des Substrates im Fermenterbehälter auf ein Niveau angehoben wird, bei dem der Gasschürzenbereich in das Substrat eintaucht. Mit einer derartigen Füllstandskontrolle wird somit auf einfache und funktionssichere Weise zuverlässig sichergestellt, dass wenigstens bei Wartungs-und Servicearbeiten der Füllstand des zu vergärenden flüssigen Substrats eine solche ausreichende Höhe aufweist, dass der starre, rohrstutzenartige Kragen in einem definierten, vorgegebenen Maße in das Substrat eintaucht, um eine zuverlässige und funktionssichere Abdichtung zur Verfügung zu stellen.

Konkret kann hierbei die Füllstands-Messvorrichtung wenigstens einen Sensor aufweisen, mittels dem der aktuelle Füllstand des Substrates im Fermenterbehälter erfasst und als Höhen-Istwertsignal der Steuereinrichtung zugeführt werden kann, die wenigstens aus diesem Höhen-Istwertsignal und dem vorgegebenen, bekannten Wert für die Einragtiefe der Gasschürze in den Fermenterbehälter-Innenraum ermittelt, ob die Gasschürze in das Substrat eintaucht. Ermittelt die Steuereinrichtung einen solchermaßen geringen Füllstand, bei dem die Gasschürze nicht in das Substrat eintaucht, wird wenigstens eine Förder- und/oder Pumpeinrichtung der Biogasanlage von der Steuereinrichtung solange angesteuert, bis der wenigstens eine Sensor ein Höhen-Istwertsignal liefert, das einen Füllstands-Sollwert und damit einem Füllstand des Substrates im Fermenterbehälter entspricht, bei dem die Gasschürze in einer gewünschten Weise in das Substrat eintaucht. Eine derartige Füllstands-Messvorrichtung mit Steuereinrichtung und Sensortechnik ist auf einfache Weise realisierbar und zudem wenig fehler- und störanfällig, was ebenfalls hilft, auf einfache und funktionssichere sowie bauteiltechnisch preiswerte Weise eine zuverlässige Abdichtung des Servicebereichs bei Wartungs- und Servicearbeiten zur Verfügung zu stellen.

Grundsätzlich gibt es unterschiedliche Möglichkeiten, den Füllstand im Fermenterbehälter ansteigen zu lassen. Beispielsweise kann hierzu die wenigstens eine Förder- und/oder Pumpeinrichtung durch wenigstens eine, das zu vergärende Substrat aus dem Fermenterbehälter abpumpende Abpumpeinrichtung gebildet sein, die zur Anhebung des Füllstandes von der Steuereinrichtung für eine definierte Zeitdauer abgeschalten wird. Eine derartige Abpumpeinrichtung kann zum Beispiel in Verbindung mit einem Nachfermenter oder dergleichen vorgesehen sein. Grundsätzlich gilt nämlich ganz allgemein, dass durch die Beschickung des Fermenters mit Substrat der Füllstand ansteigt und durch gesteuertes Abpumpen mittels einer Abpumpvorrichtung der Füllstand in etwa konstant gehalten werden kann, so dass dann mit dem Aussetzen dieses Abpumpens der Füllstand automatisch ansteigt. Alternativ oder zusätzlich dazu kann die wenigstens eine Förder- und/oder Pumpeinrichtung durch wenigstens ein, zu vergärendes flüssiges Substrat in den Fermenterbehälter pumpende bzw. förderende Pumpeinrichtung gebildet sein, die zur Anhebung des Füllstandes, gesteuert von der Steuereinrichtung, Substrat aus einem vor- und/oder nachgeschalteten Behälter in den jeweiligen Fermenterbehälter pumpt. Anstelle von flüssigem, zu vergärendem Substrat könnte grundsätzlich auch ein anderes flüssiges Medium in den Fermenterbehälter gepumpt werden, solange dies mit der Biologie der Fermentation im Fermenterbehälter-Innenraum vereinbar ist. An dieser Stelle sei ausdrücklich erwähnt, dass vorliegend unter dem Begriff zu vergärendes flüssiges Substrat jedwede zu vergärende, flüssige Biomasse bzw. Biomassemischung verstanden wird, die zur Erzeugung von Biogas einem Fermentationsprozess unterzogen werden kann.

Die Serviceöffnung selbst kann grundsätzlich auf unterschiedlichste Art und Weise ausgebildet sein, zum Beispiel rund oder mehreckig, insbesondere rechteckförmig, wobei gemäß einer besonders bevorzugten Ausgestaltung vorgesehen ist, dass die Serviceöffnung Bestandteil einer eckenseitig angeordneten, vorzugsweise begehbaren Podestplatte ist. Das heißt mit anderen Worten, dass die Serviceeinrichtung gemäß einer besonders bevorzugten Ausgestaltung zur Ausbildung einer separaten Baueinheit bzw. Baugruppe, die einfach an unterschiedlichste Fermenterbehälter-Gegebenheiten anpassbar ist, eine deckenseitig angeordnete, vorzugsweise begehbare Podestplatte aufweist, in der die Serviceöffnung ausgebildet ist. Eine derartige Podestplatte kann aus einem stabilen Material hergestellt sein, bevorzugt aus einem Metall, höchst bevorzugt aus einem Edelstahlmaterial.

In einer derartigen Podestplatte kann die Serviceöffnung auf einfache Art und Weise ausgebildet bzw. integriert werden, wobei je nach konkreten Fermenterbehälter-Gegebenheiten (zum Beispiel Foliendach oder Betondecke) eine geeignet ausgebildete Podestplatte gewählt werden kann. Insbesondere in Verbindung mit einer derartigen Podestplatte ist es besonders einfach möglich, eine die Serviceöffnung ringförmig umschließende Gasschürze auszubilden. So ist gemäß einer besonders bevorzugten Ausgestaltung vorgesehen, dass die die Serviceöffnung ringförmig umschließende Gasschürze von der Podestplatte nach unten abragt. Die Gasschürze kann durch ein separates Bauteil gebildet sein, das mit der Podestplatte verbunden ist, zum Beispiel kraft- und/oder stoffschlüssig verbunden ist. Alternativ dazu kann die Gasschürze aber auch integraler Bestandteil der Podestplatte sein und mit dieser materialeinheitlich und/oder einstückig verbunden sein, zum Beispiel durch aus der Podestplatte herausgebogene Laschen gebildet sein.

Es versteht sich, dass die Serviceöffnung und der Kragen bzw. die Gasschürze grundsätzlich unterschiedliche Geometrien aufweisen können, solange sichergestellt ist, dass die Gasschürze die Serviceöffnung vollständig umschließt. Besonders bevorzugt ist jedoch eine Ausgestaltung, bei der die Gasschürze zumindest im unmittelbaren Angrenzungsbereich an die Serviceöffnung eine der Geometrie der Serviceöffnung entsprechende Geometrie aufweist, zum Beispiel eine rechteckförmige Geometrie oder dergleichen.

Ein weiterer besonderer Vorteil einer derartigen Podestplatte ist, dass durch diese eine das Tauchgerät betätigende mediumführende oder elektrische Leitung außerhalb oder innerhalb des Gasschürzenbereiches auf einfache Weise gasdicht durch die Podestplatte hindurchgeführt werden kann. Eine derartige mediumführende bzw. elektrische Leitung weist im Fermenterbehälter-Innenraum selbstverständliche eine solche Länge auf, dass der maximale Verstellweg des Tauchgerätes dadurch nicht behindert und dementsprechend freigegeben ist.

Mit einer derartigen Podestplattenlösung mitsamt Gasschürze ergibt sich somit, wie bereits zuvor erwähnt, in Verbindung mit der als Hohlwellenanordnung ausgebildeten einzigen Lagerstelle eine einfach handhabbare und kompakt aufgebaute sowie preiswert herstellbare Serviceeinrichtung, die flexibelst an unterschiedlichste Fermenterbehälter-Gegebenheiten angepasst werden kann und sich zudem durch eine hohe Bedienfreundlichkeit auszeichnet. Besonders bevorzugt ist in diesem Zusammenhang eine Ausgestaltung, bei der die Lagerstelle der Serviceeinrichtung in der Podestplatte im an die Serviceöffnung anschließenden Podestplattenbereich (und damit im Bereich außerhalb der Serviceöffnung) angeordnet ist, insbesondere in einem der Serviceöffnung unmittelbar zugeordneten und/oder benachbarten Randbereich angeordnet ist. Damit ergibt sich eine besonders bevorzugte einfache Zuordnung des Führungsmastes mitsamt den Antrieben zur Serviceöffnung, durch die Wartungs-und Servicearbeiten am Tauchgerät durchgeführt werden können.

Die Abdeckeinrichtung für die Serviceöffnung kann grundsätzlich auch durch ein nach oben ragendes Gehäuse gebildet sein, zum Beispiel durch einen Serviceschacht entsprechend der DE 197 14 342 C1 bzw. durch ein Mastgehäuse gemäß der EP 2 174 704 A1, wobei sich hier jedoch dann, wie bereits zuvor geschildert, ein relativ großbauender und relativ bauteilintensiver und damit materialaufwändiger Aufbau ergibt. In diesem Zusammenhang könnte dann auch die Lagerstelle direkt abdeckeinrichtungsseitig am Serviceschacht bzw. am Mastgehäuse angeordnet sein. In Verbindung mit einer regelmäßig gewünschten kompakten, einfachen, wenig bauteilintensiven und damit wenig materialaufwändigen Ausführungsform einer Serviceeinrichtung, bei der hochbauende Aufbauten wie der eben erwähnte domartige Serviceschacht bzw. das ebenfalls domartige Mastgehäuse eingespart werden können, wird daher vorgeschlagen, dass diese Serviceöffnung in der Podestplatte verschließende, vorzugsweise gasdicht verschließende Abdeckeinrichtung durch wenigstens einen Deckel gebildet ist, vorzugsweise durch einen abnehmbaren oder schwenkbar angelenkten Deckel. Ein derartiger Deckel kann besonders bevorzugt plattenförmig und/oder flach ausgebildet werden, so dass hier eine zuverlässige Abdeckung und/oder Abdichtung der Serviceöffnung auch ohne den Einsatz eines nach oben wegragenden Serviceschachtes erfolgen kann. Durch die Gasschürzenanordnung ist nämlich zudem sichergestellt, dass die Service- und Wartungsarbeiten an einem in den Bereich der Serviceöffnung bewegten Tauchgerät auf funktionssichere Weise ohne nennenswerte Gasbelastung durchgeführt werden können. Insbesondere mit einem derartigen einfachen, plattenförmigen Deckel ist somit die zuvor beschriebene Ausgestaltung vorteilhaft, bei der die Lagerstelle der Serviceeinrichtung in der Podestplatte im an die Serviceöffnung anschließenden Podestplattenbereich ausgebildet bzw. angeordnet ist.

Die Podestplatte kann dabei in Verbindung mit einer im Wesentlichen horizontalen und/oder aus einem festen Material, zum Beispiel aus Beton, hergestellten Behälterdeckenwand, in eine deckenwandseitige Ausnehmung eingesetzt bzw. aufgesetzt sein. Die Anbindung kann hier zum Beispiel kraftschlüssig, zum Beispiel mittels mehrerer Schraubverbindungen erfolgen. In Verbindung mit der Anordnung einer derartigen Podestplatte in einer horizontalen Fermenterbehälter-Deckenwand ist insbesondere eine rechteckförmige Außengeometrie der Podestplatte vorteilhaft, da diese besonders einfach herzustellen ist. Zudem erweist es sich hier dann vorteilhaft, die Podestplatte in einem fermenterbehälterseitenwandnahen Bereich anzuordnen.

Gemäß einer alternativen Ausgestaltung hierzu, bei der der Biogasanlagen-Fermenterbehälter ein Foliendach aufweist, wird vorgeschlagen, dass die Podestplatte mittels einer Befestigungseinrichtung gasdicht und fest an einer Behälterwand, insbesondere einer Behälterseitenwand, des Fermenterbehälters angebunden ist, wobei die Podestplatte dann weiter einen Foliendach-Anschlussbereich aufweist, an dem das Foliendach, insbesondere ein Foliendach-Randbereich, mittelbar oder unmittelbar gasdicht angebunden ist. Die Anbindung erfolgt dabei zum Beispiel mittels Schraubverbindungen oder dergleichen. An dem Foliendach-Anschlussbereich kann insbesondere ein Foliendach-Randbereich gasdicht angebunden sein. Diese Anbindung erfolgt insbesondere mittels einer Klemmverbindung gasdicht zwischen einem Klemmbereich, insbesondere einem Randflansch der Podestplatte und einer Klemmleiste, die mit dem Klemmbereich lösbar verbindbar ist, insbesondere verklemmt ist.

Insbesondere in Verbindung mit einer Podestplattenanordnung bei einem Fermenterbehälter mit einem Foliendach hat es sich zudem als vorteilhaft erwiesen, wenn die Podestplatte in der Draufsicht in etwa die Form und/oder Außenkontur eines gleichschenkligen Trapezes aufweist und/oder die Serviceöffnung sowie der Deckel in etwa in einem mittleren Bereich der Podestplatte angeordnet sind.

Für eine besonders stabile Abstützung der Podestplatte bei einer Foliendachanordnung an einem Biogasanlagen-Fermenterbehälter kann zudem eine Abstützeinrichtung vorgesehen sein, die zum Beispiel in der Art eines Abstützgestells ausgebildet ist, die die Podestplatte von unten her abstützt und am Fermenterbehälter-Innenraumwandbereich angebunden und festgelegt ist. Hierbei ist zudem gemäß einer besonders bevorzugten weiteren Ausgestaltung vorgesehen, dass die Gasschürze so zwischen zwei beabstandeten Anschlussstützen der Abstützeinrichtung angeordnet ist, dass die Anschlussstützen die rechteckförmig ausgebildete Gasschürze auf gegenüberliegenden Rechtecklängsseiten der Gasschürze abstützen. Dadurch wird auf zuverlässige Weise eine Beeinträchtigung der Gasschürze durch sich eventuell aufbauenden hohen Druck vermieden.

Ferner wird mit den Merkmalen des Patentanspruchs 18 eine Serviceeinrichtung zur Verwendung in einem Biogasanlagen-Fermenterbehälter beansprucht, mit dem sich die zuvor genannten Vorteile ergeben, so dass auf die Wiederholung dieser Vorteile hier aus Übersichtlichkeitsgründen und zur Vermeidung von Wiederholungen verzichtet wird.

Die Erfindung wird nachfolgend anhand einer Zeichnung näher erläutert.

Es zeigen:
- Fig. 1: schematisch einen Querschnitt durch einen randseitigen Teilbereich eines Biogasanlagen-Fermenterbehälters mit einem Foliendach,
- Fig. 2: schematisch und perspektivisch eine Darstellung einer trapezartigen Podestplatte mitsamt Serviceöffnung, Serviceöffnungsdeckel, Gasschürze und einziger Lagerstelle,
- Fig. 3: schematisch eine Ansicht der Podestplatte entsprechend des Pfeils A der Fig. 2,
- Fig. 4a: schematisch eine Seitenansicht eines oberen Randbereichs einer Seitenwand eines Fermenterbehälters vor dem Ansetzen der Podestplatte gemäß Fig. 2 und Fig. 3,
- Fig. 4b: die Podestplatte gemäß. Fig. 2 und Fig. 3 im montierten Zustand in einer Ansicht entsprechend des Pfeils B der Fig. 3,
- Fig. 5: eine schematisch Rückansicht entsprechend des Pfeils C der Fig. 3 (mitsamt einem hier lediglich beispielhaft dargestellten Antriebsmotor),
- Fig. 6: schematisch und beispielhaft eine vergrößerte Detaildarstellung der podestplattenseitigen Hohlwellenanordnung der Fig. 1,
- Fig. 7: eine alternative Ausführungsform einer podestplattenseitigen Hohlwellenanordnung mit manuellem Schwenkantrieb und manueller Höhenverstelleinrichtung, und
- Fig. 8: schematisch und beispielhaft eine Ansicht in Richtung des Pfeils D der Fig. 7.

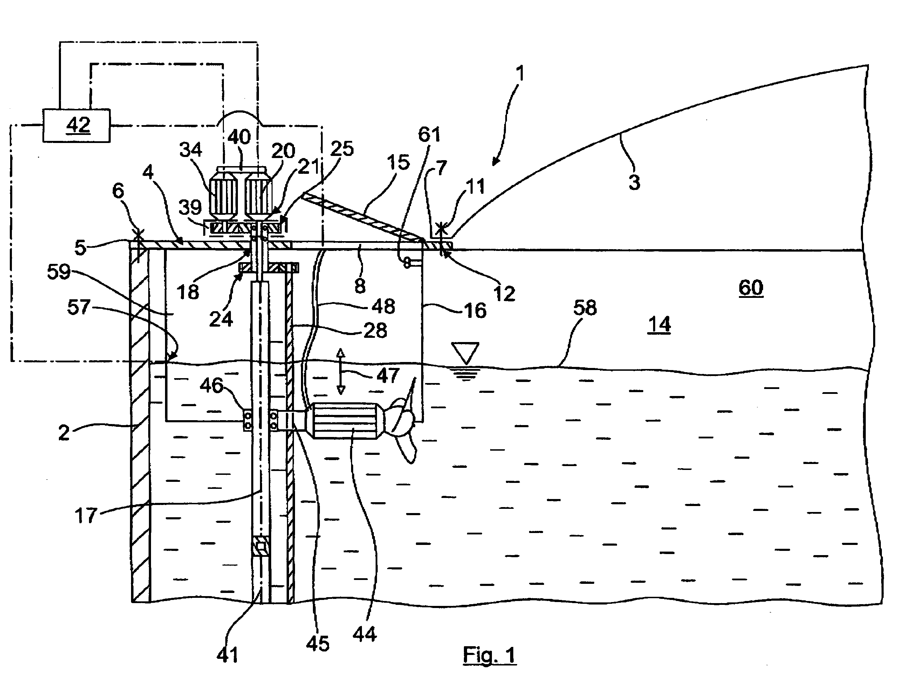

In der Fig. 1 ist schematisch ein Querschnitt durch einen Teilbereich einer beispielhaften Ausführungsform eines erfindungsgemäßen Biogasanlagen-Fermenterbehälters 1 gezeigt, der sowohl ein Vor-, als auch ein Haupt-, als auch ein Nach-Fermenterbehälter sein kann.

Dieser Fermenterbehälter weist eine hier nicht gezeigte Bodenwand sowie eine beispielsweise kreiszylindrische Seitenwand 2 auf und ist an seiner Oberseite und damit deckenseitig mit einem hier lediglich schematisch und prinzipiell dargestellten Foliendach 3 abgedeckt. Insofern ist der Fermenterbehälter 1 herkömmlicher Bauart.

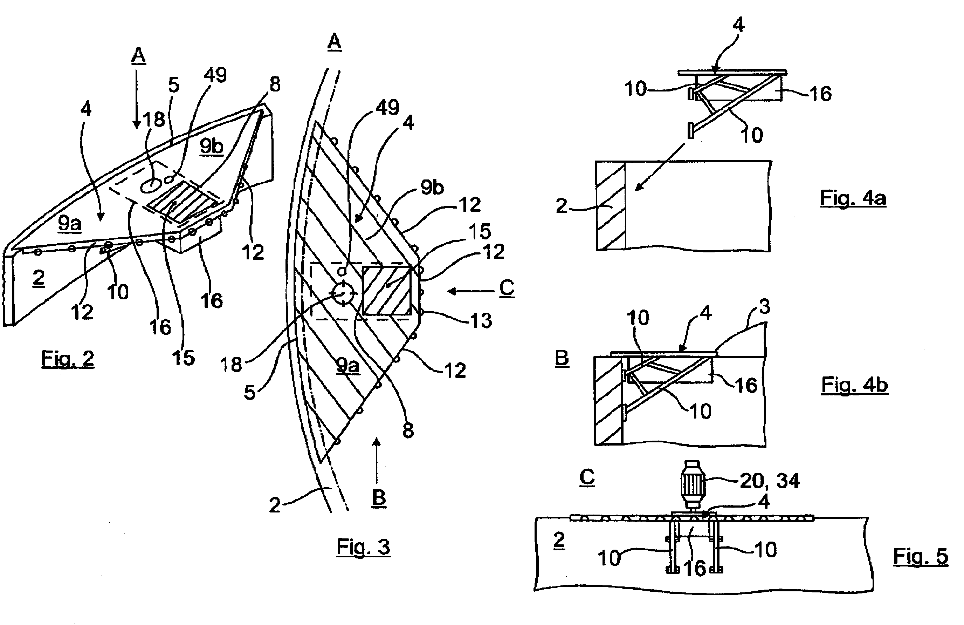

In der hier in der Fig. 1 gezeigten Ausgestaltung ist an einem oberen Fermenterbehälterrandbereich, der Seitenwand 2 zugeordnet, eine eine Montageplatte ausbildende Podestplatte 4 angeordnet, die, wie dies insbesondere den Fig. 2 und 3 zu entnehmen ist, einen der Seitenwand 2 zugeordneten Podestplatten-Randkantenbereich 5 aufweist, mittels dem die Podestplatte 4 hier beispielshaft von oben her auf der Seitenwand 2 aufliegt und dort mittels der hier lediglich schematisch dargestellten Schraubverbindungen 6 lösbar festgelegt ist. Zur Herstellung der Gasdichtheit kann hier zum Beispiel ein nicht gezeigtes Dichtelement im Auflagebereich des Podestplatten-Randkantenbereichs 5 auf der Seitenwand 2 angeordnet und vorgesehen sein.

Wie dies insbesondere aus den Fig. 2 und 3 in Zusammenschau mit der Fig. 1 ersichtlich ist, weist die hier in der Form eines gleichschenkligen Trapezes ausgebildete Podestplatte 4 in etwa mittig und zentral eine hier beispielhaft rechteckförmige Serviceöffnung 8 auf, an die sich zu beiden Seiten hin flügelhafte Podestplattenbereiche 9a, 9b anschließen. An diesen flügelartigen Podestplattenbereichen 9a, 9b greifen beispielsweise insbesondere aus der Fig. 4a, 4b und Fig. 5 ersichtliche Stützen 10 eines Abstützgestells von unten her an, welche Stützen 10 weiter am stabilen Seitenwandbereich 2 fest angebunden sind, sodass die Stützen 10 die Podestplatte 4 in der, in der Fig. 4b gezeigten Position halten.

Auf der dem Podestplatten-Randkantenbereich 5 gegenüberliegenden Seite der Podestplatte 4 ist, hier lediglich in der Fig. 1 schematisch und beispielhaft dargestellt, das Foliendach 3, insbesondere ein Foliendach-Randbereich 7 mittels mehrerer kraftschlüssiger Verbindungen 11 gasdicht angebunden. Die Anbindung kann zum Beispiel über Klemmleisten oder dergleichen erfolgen, zwischen denen und der Podestplatte 4 das Foliendach gasdicht verklemmt ist.

In dem Foliendach-Anschlussbereich 12 ist ferner, was insbesondere aus der Fig. 2 und der Fig. 3 ersichtlich ist, eine Mehrzahl von voneinander in Längsrichtung beabstandeten Ösen 13 angeordnet, durch die zum Beispiel hier nicht dargestellte Seile zum Festlegen beziehungsweise Abspannen des Foliendaches hindurch gefädelt und/oder festgebunden werden können.

Wie dies weiter insbesondere der Zusammenschau der Fig. 1 bis 3 entnommen werden kann, ist um die Serviceöffnung 8 herum eine starre, im montieren Zustand der Podestplatte 4 schachtartig nach unten in den Fermenterbehälter-Innenraum 14 einragende, nicht verlagerbare und unflexible rohrstützenartige Gasschürze 16 angeordnet, die zum Beispiel durch ein separates Bauteil aus einem zum Beispiel Edelstahlmaterial hergestellt sein kann und dann mit der Podestplatte 4 verschraubt beziehungsweise verschweißt sein kann.

Wie dies insbesondere wiederum aus der Zusammenschau der Fig. 1 bis 3 ersichtlich ist, ist die in der Podestplatte 4 ausgebildete Serviceöffnung 8 mittels eines podestplattenseitig schwenkbar angelenkten beziehungsweise gegebenenfalls auch vollständig abnehmbaren Deckels 15 vorzugsweise gasdicht verschließbar. Dieser Deckel 15 ist bevorzugt so ausgebildet, dass er sich im Wesentlichen oberflächenbündig in die Podestplatte 4 einschmiegt, sodass eine im Wesentlichen glatte und ebene durchgehende beziehungsweise begehbare Podestplatte 4 ausgebildet ist. Es versteht sich, dass selbstverständlich auch die Podestplatte aus einem stabilen Material hergestellt ist, zum Beispiel einem Metall, insbesondere einem Edelstahlmaterial.

Wie dies aus der Fig. 1 weiter ersichtlich ist, ist im Fermenterbehälter 1 ein hier beispielhaft durch ein Quadratleitrohr gebildeter Führungsmast 17 angeordnet, der in einem hier nicht dargestellten bodenseitigen Stützlager verdrehbar gelagert ist. Dieser Führungsmast 17 ist in noch zu beschreibender Weise in einer podestplattenseitigen Lagerstelle 18 der Podestplatte 4 unmittelbar benachbart zur Serviceöffnung 8 gelagert, sodass die Lagerstelle 18 ebenfalls im von der Gasschürze 16 umgrenzten Podestplattenbereich liegt.

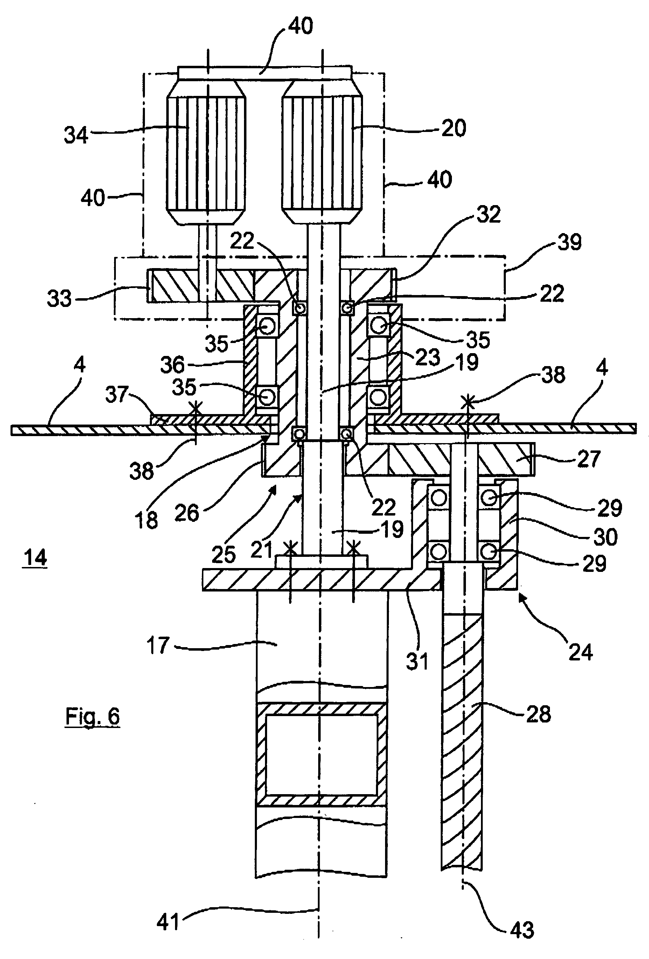

Wie dies nunmehr insbesondere durch die Zusammenschau der Fig. 1 mit der Fig. 6 ersichtlich ist, ist der durch ein Quadratleitrohr ausgebildete Führungsmast 17 mit einer Lagerwelle 19 durch die podestplattenseitige Lagerstelle 18 hindurchgeführt, wobei die Lagerwelle 19 gleichzeitig eine Abtriebswelle eines elektrischen Antriebsmotors 20 einer Führungsmast-Schwenkeinrichtung 21 ausbildet.

Wie dies insbesondere der Fig. 6 entnommen werden kann, ist die Lagerwelle 19 über Kugellager 22 verdrehbar in einer als Hohlwelle ausgebildeten Antriebswelle 23 einer Tauchgerät-Höhenverstelleinrichtung 24 verdrehbar aufgenommen und gelagert, welche Antriebswelle 23 zusammen mit der Lagerwelle eine Hohlwellenanordnung 25 ausbildet, die in der podestplattenseitgen Lagerstelle 18 aufgenommen ist.

Wie dies der Fig. 6 weiter entnommen werden kann, weist die Antriebswelle 23 im Fermenterbehälter-Innenraum 14 und damit in der Bildebene der Fig. 6 unterhalb der Podestplatte 4 ein Antriebszahnrad 26 auf, das hier beispielhaft unmittelbar mit einem Gewindespindel-Zahnrad 27 einer Gewindespindel 28 kämmt.

Wie dies insbesondere auch der Fig. 1 entnommen werden kann, erstreckt sich diese Gewindespindel 28 in etwa parallel zum Führungsmast 17 und ist an diesem sowohl kopfseitig, das heißt podestplattenseitig, als auch bodenseitig (nicht dargestellt) verdrehbar gelagert. Diese drehbare Lagerung erfolgt hier beispielhaft über Kugellager 29, die in einer Lageraufnahme 30 eines mit dem Führungsmast 17 fest verbundenen Stützarms 31 für die Gewindespindel 28 aufgenommen und angeordnet sind.

Auf der dem Antriebszahnrad 26 der Antriebswelle 23 gegenüberliegenden Seite, die außerhalb des Fermenterbehälter-Innenraums 14 und damit oberhalb der Podestplatte 4 liegt, weist das Antriebszahnrad 26 hier beispielhaft ein weiteres äußeres Zahnrad 32 auf, das mit einem Antriebsritzel 33 eines weiteren elektrischen Antriebsmotors 34 kämmt.

Wie dies der Fig. 6 zudem weiter entnommen werden kann, ist die als Hohlwelle ausgebildete Antriebswelle 23 zudem über Kugellager 35 in einer der Lagerstelle 18 der Podestplatte 4 zugeordneten gehäuseartigen Hülse 36 verdrehbar beziehungsweise verschwenkbar gelagert, wobei die Hülse hier ferner einen Befestigungsflansch 37 aufweist, mittels dem die Hülse um die Lagerstelle 18 der Podestplatte 4 herum mittels hier beispielhaft dargestellter Schraubverbindungen 38 gegebenenfalls unter Zwischenschaltung einer Dichtung, fest und unverdrehbar mit der Podestplatte 4 verbunden ist.

Wie dies der Fig. 6 weiter lediglich äußerst schematisch und strichliert zu entnehmen ist, können das Antriebsritzel 33 sowie das äußere Zahnrad 32 in einem Schutzgehäuse 39 aufgenommen sein, das wiederum mit der freien Oberseite der Hülse 36 verbunden sein kann, sodass eine insgesamt dichte Anordnung der Hohlwellenanordnung 25 an beziehungsweise in der Lagerstelle 18 erfolgen kann. In diesem Zusammenhang ist dann lediglich sicher zu stellen, dass die Antriebswellen der beiden Antriebsmotoren 20 beziehungsweise 34 dicht durch das Schutzgehäuse 39 herausgeführt sind. Wie in der Fig. 6 weiter schematisch angedeutet, können die beiden Antriebsmotoren 20 beziehungsweise 34 ferner über eine Stütz- und/oder Gestellanordnung 40 miteinander und/oder mit der Hülse 36 fest verbunden sein.

Soll nunmehr beispielsweise der Führungsmast 17 um die vertikale Schwenkachse 41 verschwenkt werden, so kann mittels der in der Fig. 1 lediglich beispielhaft und schematisch eingezeichneten Steuer und/oder Regeleinrichtung, in Abhängigkeit von vorgegebenen Steuer- beziehungsweise Regelparamtern der Antriebsmotor 20 angesteuert werden und die Lagerwelle 19 so weit um die Schwenkachse 41 verschwenkt werden, bis sich der Führungsmast 17 in der gewünschten Schwenkposition befindet. Durch die Kugellager 22 in Verbindung mit der Hohlwellenanordnung 25 ist dabei sichergestellt, dass sich die Lagerwelle 19 relativ zur als Hohlwelle ausgebildeten Antriebswelle 23 verdrehen beziehungsweise verschwenken kann. Da allerdings die Gewindespindel 28 über den Stützarm 31 fest mit dem Führungsmast 17 verbunden ist, muss bei einer derartigen Schwenkbewegung des Führungsmastes um die Schwenkachse 41 auch sicher gestellt sein, dass die Gewindespindel 28 entsprechend mit verschwenkt werden kann. Dies ist durch die drehbare Anordnung der als Hohlwelle ausgebildeten Antriebswelle 23 der Hohlwellenanordnung 25 beziehungsweise durch die Zahnradpaarung 26, 27 sichergestellt.

Andererseits kann bei einer Betätigung der Tauchgerät-Höhenverstelleinrichtung 24 mit der Steuer- und/oder Regeleinrichtung 42 der Antriebsmotor 34 entsprechend vorgegebener Steuer- beziehungsweise Regelparamter angesteuert werden, wodurch das Antriebsritzel 33 die Antriebswelle 23 antreibt, die wiederum aufgrund der Kugellager 22 und 35 sowohl relativ zur Hülse 36 als auch relativ zur Lagerwelle 19 verdrehbar ist und somit das Antriebszahnrad 26 das Gewindespindel-Zahnrad 27 antreibt, wodurch wiederum die Gewindespindel 28 um die Spindelachse 43 verdreht wird.

Wie dies in der Fig. 1 lediglich äußerst schematisch dargestellt ist, durchgreift die Gewindespindel 28 eine an einem Tauchmotor-Rührgerät 44 angeordnete Spindelmutter 45, die Bestandteil eines Führungsschlittens 46 ist, mittels dem das Tauchmotor-Rührgerät 44 entlang des Führungsmastes 17 verfahrbar ist.

Je nach der Drehrichtung der Gewindespindel 28 wird dabei das Tauchmotor-Rührgerät 44, wie in der Fig. 1 durch den Doppelpfeil 47 gezeigt, verfahren, zum Beispiel für Wartungs- und Servicearbeiten nach oben in Richtung Podestplatte 4 und damit in die Gasschürze 16 hinein verfahren, und zwar bevorzugt so weit, dass das Tauchmotor-Rührgerät 44 unmittelbar unterhalb der Serviceöffnung 8 zum Liegen kommt, sodass das Tauchmotor-Rührgerät 44 durch entsprechendes Öffnen des Deckels 15, wie in der Fig. 1 beispielhaft schematisch gezeigt, für Wartungs- und Servicearbeiten von der Podestplatte 4 aus zugänglich ist.

Die Wartungs-und Servicearbeiten können dabei vom Wartungs- und Servicepersonal direkt am serviceöffnungsseitig angeordneten Tauchmodul-Rührgerät 44 durchgeführt werden. Alternativ dazu ist es aber auch möglich, das Tauchmotor-Rührgerät 44 vom Führungsschlitten 46 abzumontieren und das Tauchmotor-Rührgerät 44 insgesamt aus dem Fermenterbehälter 1 zu entnehmen. Nach durchgeführten Wartungs- und Servicearbeiten beziehungsweise auch nach dem Austausch eines Tauchmotor-Rührgeräts 44 kann dieses beziehungsweise ein neues Gerät wiederum am Führungsschlitten 46 befestigt werden.

Wie dies in der Fig. 1 zudem weiter dargestellt ist, kann eine das Tauchmotor-Rührgerät 44 betätigende hier beispielhaft elektrische Leitung 48 gasdicht durch die Podestplatte 4 hindurch geführt werden, insbesondere an der in der Fig. 2 und 3 gezeigten Durchführstelle 49 im Bereich der Gasschürze gasdicht hindurchgeführt werden, und z.B. als Signalleitung auch entsprechend mit der Steuer- und/oder Regeleinrichtung 42 steuer- beziehungsweise regelungstechnisch gekoppelt sein. Eine derartige Durchführung im Bereich der Gasschürze ist vorteilhaft, da ansonsten das Rührgerät nicht entnommen werden kann, ohne die elektrische Leitung 48 vorher abzumontieren. Es versteht sich, dass die elektrische Leitung 48 selbstverständlich eine solche Länge aufweisen muss, dass das Verstellen des Tauchmotor-Rührgeräts 44 über die gesamte Verstelllänge der Gewindespindel 28 ungehindert erfolgen kann.

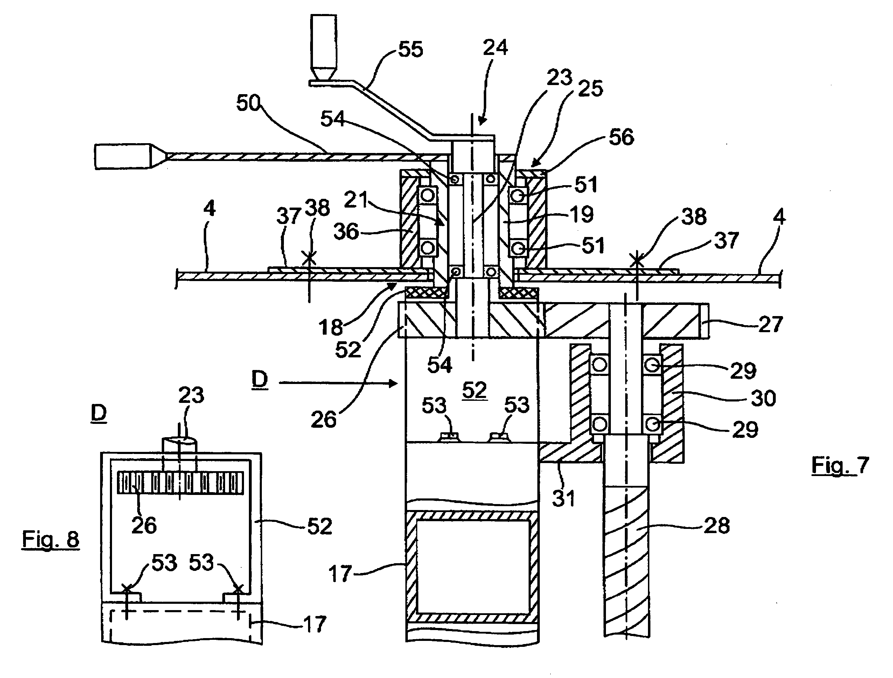

In der Fig. 7 ist eine alternative Ausführungsform der Hohlwellenanordnung 25 gezeigt, bei der nunmehr die Lagerwelle 19 der Führungsmast-Schwenkeinrichtung 21 als Hohlwelle ausgebildet ist und mit einem handbetätigbaren Schwenkhebel 50 verbunden ist. Die Lagerwelle 19 ist hier über entsprechende Kugellager 51 in der Hülse 36 verdrehbar gelagert, die über den Befestigungsflansch 37 mitsamt Befestigungsschrauben 38 um die Lagerstelle 18 herum an der Podestplatte 4 festgelegt und unverdrehbar angebunden ist.

Wie dies insbesondere aus der Zusammenschau der Fig. 7 und 8 ersichtlich ist, ist die Lagerwelle 19 mit einer hier beispielhaft U-förmigen Lagerbrücke 52 fest verbunden, die wiederum, wie in der Fig. 8 dargestellt, mit dem Führungsmast 17 fest verbunden ist. Die Anbindung der Lagerbrücke am Führungsmast 17 erfolgt beispielhaft mittels Schraubverbindungen 53. Ebenso kann die Anbindung der Lagerwelle 19, die hier als Hohlwelle ausgebildet ist, an der Lagerbrücke 52 kraftschlüssig, das heißt mittels Schraubverbindungen erfolgen. Auch eine stoffschlüssige Anbindung ist selbstverständlich möglich.

Durch die als Hohlwelle ausgebildete Lagerwelle 19 hindurch ist hier die Antriebswelle 23 der Tauchgerät-Höhenverstelleinrichtung 24 geführt, die über Kugellager 54 relativ zur Lagerwelle 19 verdrehbar in dieser aufgenommen ist. Für eine Handbetätigung ist auch die Antriebswelle 23 hier mittels einer Handkurbel 55 fest verbunden. Bei einer entsprechenden Drehbetätigung der Handkurbel 55 kann somit die Antriebswelle 23 in der Hohlwellenanordnung 25 verdreht werden, wodurch das Antriebszahnrad 26 der Antriebswelle 23 das Gewindespindel-Zahnrad 27 und damit die Gewindespindel 28 in der zuvor beschriebenen Weise antreibt. Auch hier ist wiederum analog zur Ausgestaltung nach Fig. 6 die Gewindespindel 28 über Kugellager 29 in einer Lageraufnahme 30 eines Stützarms 31, der mit dem Führungsmast 17 fest verbunden ist, verdrehbar gelagert.

Wie dies weiter insbesondere aus der Fig. 8 ersichtlich ist, ist die Lagerbrücke 52 und/oder das Antriebszahnrad 26 hier so dimensioniert, dass die Bewegung des Antriebszahnrads 26 berührungsfrei zur Umgebung erfolgt und damit die Höhenverstellung des Tauchmotor-Rührgeräts 44 entlang der Gewindespindel 28 nicht beeinträchtigt wird.

Wie der Fig. 7 zudem weiter entnommen werden kann, ist hier die Hülse 36 am oberen freien Ende zudem mittels eines Schutzdeckels 56 abgedeckt, um eine Dichtheit der Anordnung sicher zu stellen.

Ansonsten entspricht die Funktionsweise der Führungsmast-Schwenkeinrichtung 21 beziehungsweise der Tauchgerät-Höhenverstelleinrichtung derjenigen, wie sie zuvor in Verbindung mit der Ausführungsform nach Fig. 6 näher erläutert worden ist, sodass auf eine diesbezügliche Wiederholung verzichtet wird.

An dieser Stelle sei lediglich weiter erwähnt, dass selbstverständlich auch die Ausführungsform nach Fig. 6 anstelle der Antriebsmotoren 20 und 34 handbetätigt mittels eines Schwenkhebels beziehungsweise einer Handkurbel erfolgen kann, wie sie in der Fig. 7 beispielhaft gezeigt sind. Alternativ kann auch die Handkurbel beziehungsweise der Schwenkhebel der Ausführungsform nach Fig. 7 jeweils durch entsprechende elektrische hydraulische und/oder pneumatische Antriebsmotoren ersetzt werden. Auch eine Kombination von Antriebsmotor und Handbetätigungselement ist selbstverständlich jederzeit möglich. Insofern geben die beispielhaften Ausführungsformen der Fig. 6 und 7 lediglich beispielhafte Ausführungsformen wieder.

Um zu verhindern, dass bei einer Öffnung des Deckels 15 Gas über die Serviceöffnung 8 in die Umgebung entweichen kann, ist in der Gasschürze 16 weiter wenigstens ein in der Fig. 1 lediglich äußerst schematisch und beispielhaft dargestellter Sensor 57 als Bestandteil einer Füllstands-Messeinrichtung vorgesehen. Mittels dieses wenigstens einen Sensors 57 kann die Füllstandshöhe des Substrates 58 im Fermenterbehälter-Inneraum 14 messtechnisch erfasst und als Höhen-Istwertsignal der Steuer- und/oder Regeleinrichtung 42 zugeführt werden, die bei einem im Beispielfall der Fig. 1 erfassten Füllstand des Substrates 58, bei dem die Gasschürze 16 in das Substrat 58 eintaucht ein Signal ausgibt, dass der Substratfüllstand in Ordnung ist und dementsprechend der Deckel 15 geöffnet werden kann, sowie das Tauchmotor-Rührgerät 44 nach oben zur Serviceöffnung 8 verlagert werden kann, wie dies zuvor bereits ausführlich gewürdigt worden ist. In diesem Fall kann dann zum Beispiel eine Pumpeinrichtung gestoppt werden, die den Substratfüllstand 58 im Fermenterbehälter-Innenraum 14 hat ansteigen lassen, um sicher zu stellen, dass eine Gasphase 59 in der Gasschürze 16 von einer Gasphase 60 im Fermenterbehälter 1 abgetrennt ist. Gegebenenfalls kann auch noch eine Absaugeinrichtung vorgesehen sein, die vor dem Öffnen des Deckels 15 die Gasphase 59 in der Gasschürze 16 absaugt, was hier allerdings nicht dargestellt ist.

Mit anderen Worten bedeutet dies, dass im Falle eines Substrat-Füllstandes im Fermenterbehälter 1, bei dem die Gasschürze 16 mit einem unteren freien Randbereich nicht in das Substrat 58 eintaucht, die Öffnung des Deckels 15 und damit der Serviceöffnung 8 nicht freigegeben wird und in diesem Fall dann über eine entsprechende Pumpeinrichtung so lange Substrat beziehungsweise andere geeignete Flüssigkeit nachgepumpt wird, bis der Substratfüllstand auf die gewünscht Höhe ansteigt.

Nach Beendigung der Service- beziehungsweise Wartungsarbeiten kann dann das Tauchmotor-Rührgerät 44 wieder über die Serviceöffnung 8 in den Innenraum 14 des Fermenterbehälters bewegt werden und anschließend der Deckel 15 und damit die Serviceöffnung 8 wiederum gasdicht verschlossen werden.

Anschließend kann dann weiter die Steuereinrichtung 42 ein Abpumpen von flüssigem Substrat 58 einleiten, wodurch sich der Füllstand im Fermenterbehälter-Innenraum bevorzugt auf ein Niveau absenkt, in dem die Gasschürze 16 nicht in das Substrat 58 eintaucht, wodurch sichergestellt ist, dass wiederum eine einheitliche Gasphase 60 oberhalb des Flüssigkeitsspiegels des Substrats vorhanden ist.

Wie dies insbesondere der Fig. 1 entnommen werden kann, ist, vorzugsweise im Bereich des Deckels 15 und damit von außen bei geöffnetem Deckel 15 gut zugänglich, ein verschließbarer Auslass, insbesondere ein Gasauslass 61, innenwandseitig an der Gasschürze 16 angeordnet. Bei in die Flüssigphase eintauchender Gasschürze 16 kann dann, z.B. in bestimmten Notfallsituationen, z.B. in solchen, in denen der Füllstand sehr stark ansteigt, zuerst der Deckel 15 geöffnet werden, dann anschließend der Gasauslass 61 geöffnet werden und so Gas gezielt aus dem Fermenterbehälter-Innenraum 14 über die Gasschürze 16 nach außerhalb des Fermenters abgeleitet bzw. abgezogen werden.

## Patentansprüche

1. Biogasanlagen-Fermenterbehälter mit einer Serviceeinrichtung, die eine mittels einer Abdeckeinrichtung (15) verschließbare deckenseitige Serviceöffnung (8) aufweist, durch die ein an einem Führungsmast (17) höhenverstellbar geführtes Tauchgerät (44) für Wartungs- und Servicearbeiten zugänglich ist,
wobei der Führungsmast (17) mittels einer Lagerwelle (19) verdrehbar und/oder verschwenkbar in einer Lagerstelle (18) der Serviceeinrichtung gelagert ist, wobei die Lagerwelle (19) zum Verdrehen oder Verschwenken des Führungsmastes (17) mittels einer außerhalb des Fermenterbehälters (1) angeordneten Führungsmast-Schwenkeinrichtung (21) gekoppelt ist,
wobei das Tauchgerät (44) mit einer außerhalb des Fermenterbehälters (1) angeordneten Tauchgerät-Höhenverstelleinrichtung (24) gekoppelt ist, die eine Antriebswelle (23) aufweist, die zusammen mit der Lagerwelle (19) der Führungsmast-Schwenkeinrichtung in einer Hohlwellenanordnung (25) durch die Lagerstelle (18) der Serviceeinrichtung hindurch in den Fermenterbehälter-Innenraum (14) geführt ist, und
wobei die Antriebswelle (23) im Fermenterbehälter-Innenraum (14) mittelbar oder unmittelbar mit einem Verstellelement (28) zusammenwirkt, mittels dem das Tauchgerät (44) am Führungsmast (17) höhenverstellbar ist.

2. Biogasanlagen-Fermenterbehälter nach Anspruch 1, **dadurch gekennzeichnet, dass** das Verstellelement durch eine sich parallel zum Führungsmast (17) erstreckende und dort drehbar gelagerte Gewindespindel (28) gebildet ist, wobei die Gewindespindel (28) eine tauchgeräteseitige Spindelmuttereinrichtung (45) so durchgreift, dass das Tauchgerät (44) bei einer Drehbetätigung der Gewindespindel (28) mittels der Antriebswelle, in Abhängigkeit von der Drehrichtung der Gewindespindel, entlang des Führungsmastes (17) nach oben oder nach unten verlagerbar ist.

3. Biogasanlagen-Fermenterbehälter nach Anspruch 1 oder 2, **dadurch gekennzeichnet, dass** die Hohlwellenanordnung (25) so ausgebildet ist, dass entweder die Lagerwelle (19) der Führungsmast-Schwenkeinrichtung (21) oder die Antriebswelle (23) der Tauchgerät-Höhenverstelleinrichtung (24) als Hohlwelle ausgebildet ist, die an und/oder in der Lagerstelle (18) der Serviceeinrichtung verdrehbar gelagert ist, insbesondere gleit- oder kugelgelagert ist, und in der die jeweils andere Welle verdrehbar aufgenommen und gelagert ist, insbesondere gleit- oder kugelgelagert ist.

4. Biogasanlagen-Fermenterbehälter nach einem der vorhergehenden Ansprüche, **dadurch gekennzeichnet, dass** die Antriebswelle (23) der Tauchgerät-Höhenverstelleinrichtung (24) im Fermenterbehälter-Innenraum (14) ein Antriebswellen-Antriebselement (26), insbesondere ein Antriebszahnrad, aufweist, das mittelbar oder unmittelbar mit einem mit dem Verstellelement (28), insbesondere einer Gewindespindel, mittelbar oder unmittelbar gekoppelten Verstellelement-Antriebselement (27), insbesondere einem Gewindespindel-Antriebselement, bevorzugt einem Gewindespindel-Zahnrad, zusammenwirkt.

5. Biogasanlagen-Fermenterbehälter nach Anspruch 3 und 4, **dadurch gekennzeichnet, dass** die Lagerwelle (19) der Führungsmast-Schwenkeinrichtung (21) als Hohlwelle ausgebildet ist, durch die die Antriebswelle (23) der Tauchgerät-Höhenverstelleinrichtung (24) in den Fermenterbehälter-Innenraum (14) geführt ist, wobei die Lagerwelle (19) mit einer mit dem Führungsmast (17) unmittelbar oder mittelbar verbundenen Lagerbrücke (52) gekoppelt ist, die das Antriebswellen-Antriebselement (26) berührungsfrei überbrückt und/oder in einem brückenseitigen Frei- oder Aufnahmeraum aufnimmt, oder dass alternativ die Antriebswelle (23) der Tauchgerät-Höhenverstelleinrichtung (24) als das Antriebswellen-Antriebselement (26) aufweisende Hohlwelle ausgebildet ist, durch die die Lagerwelle (19) der Führungsmast-Schwenkeinrichtung (21) in den Fermenterbehälter-Innenraum (14) geführt ist.

6. Biogasanlagen-Fermenterbehälter nach einem der vorhergehenden Ansprüche, **dadurch gekennzeichnet, dass** die Führungsmast-Schwenkeinrichtung (21) durch einen mit der Lagerwelle (19) gekoppelten handbetätigbaren Schwenkhebel (50) oder durch einen Antriebsmotor (20) gebildet ist, wobei eine Abtriebswelle des Antriebsmotors (20) die Lagerwelle (19) der Führungsmast-Schwenkeinrichtung (21) ausbildet oder Bestandteil der Lagerwelle (19) der Führungsmast-Schwenkeinrichtung ist oder mit der Lagerwelle der Führungsmast-Schwenkeinrichtung (21) unmittelbar oder mittelbar drehübertragend gekoppelt ist.

7. Biogasanlagen-Fermenterbehälter nach einem der vorhergehenden Ansprüche, **dadurch gekennzeichnet, dass** die Tauchgerät-Höhenverstelleinrichtung (24) durch eine mit der Antriebswelle (23) gekoppelte handbetätigbare Kurbeleinrichtung (55) oder durch einen Antriebsmotor (34) gebildet ist, wobei eine Abtriebswelle des Antriebsmotors (34) die Antriebswelle (23) der Tauchgerät-Höhenverstelleinrichtung (24) ausbildet oder Bestandteil der Antriebswelle (23) der Tauchgerät-Höhenverstelleinrichtung (24) ist oder mit der Antriebswelle (23) der Tauchgerät-Höhenverstelleinrichtung (24) unmittelbar oder mittelbar bewegungsübertragend gekoppelt ist.

8. Biogasanlagen-Fermenterbehälter nach einem der vorhergehenden Ansprüche, **dadurch gekennzeichnet, dass** die Hohlwellenanordnung (25) so ausgebildet ist, dass die Hohlwelle in einer der Lagerstelle (18) der Serviceeinrichtung zugeordneten Hülse (36) drehbar und/oder verschwenkbar gelagert ist, insbesondere gleit- oder kugelgelagert ist, wobei die Hülse (36) ferner einen Befestigungsflansch (37) aufweist, mittels dem die Hülse (36) um die Lagerstelle (18) der Serviceeinrichtung herum, insbesondere um die Lagerstelle (18) einer die Serviceöffnung (8) aufweisenden Podestplatte (4) herum, fest und unverdrehbar mit der Serviceeinrichtung, insbesondere der Podestplatte (4), verbunden ist.

9. Biogasanlagen-Fermenterbehälter nach einem der vorhergehenden Ansprüche, **dadurch gekennzeichnet, dass** um die Serviceöffnung (8) herum eine in der Dichtstellung schachtartig nach unten in den Fermenterbehälter-Innenraum (14) einragende Gasschürze (16) angeordnet ist, die wenigstens bei Wartungs- und Servicearbeiten in das im Fermenterbehälter (1) aufgenommene, zu vergärende flüssige Substrat eintaucht und den von der Gasschürze (16) ringförmig umschlossenen Gasphasenbereich (59) unterhalb der öffenbaren Serviceöffnung (8) sowie oberhalb des Substrates (58) gasdicht von dem restlichen Gasphasenbereich (60) des Fermenterbehälter-Innenraums (14) abtrennt, wobei bevorzugt vorgesehen ist, dass die Gasschürze (16) durch einen starren, nicht verlagerbaren und unflexiblen sowie von der Serviceöffnung (8) dauerhaft in Richtung Fermenterbehälter-Innenraum (14) abragenden rohrstutzenartigen Kragen gebildet ist.

10. Biogasanlagen-Fermenterbehälter nach einem der vorhergehenden Ansprüche, **dadurch gekennzeichnet, dass** die Serviceeinrichtung eine deckenseitig angeordnete, vorzugsweise begehbare Podestplatte (4) ist oder aufweist, in der die Serviceöffnung (8) ausgebildet ist.

11. Biogasanlagen-Fermenterbehälter nach Anspruch 9 und 10, **dadurch gekennzeichnet, dass** die die Serviceöffnung (8) ringförmig umschliessende Gasschürze (16) von der Podestplatte (4) nach unten abragt.

12. Biogasanlagen-Fermenterbehälter nach Anspruch 11, **dadurch gekennzeichnet, dass** die Gasschürze (16) durch ein separates Bauteil gebildet ist, das mit der Podestplatte (4) verbunden ist, insbesondere kraft- und/oder stoffschlüssig verbunden ist, oder dass die Gasschürze (16) integraler Bestandteil der Podestplatte (4) ist und mit dieser materialeinheitlich und/oder einstückig verbunden ist, insbesondere durch aus der Podestplatte (4) herausgebogene Laschen gebildet ist.

13. Biogasanlagen-Fermenterbehälter nach einem der Ansprüche 10 bis 12, **dadurch gekennzeichnet, dass** die die Serviceöffnung (8) in der Podestplatte (4) verschließende, vorzugsweise gasdicht verschließende Abdeckeinrichtung durch wenigstens einen Deckel (15) gebildet ist.

14. Biogasanlagen-Fermenterbehälter nach einem der Ansprüche 10 bis 13, **dadurch gekennzeichnet, dass** die Lagerstelle (18) der Serviceeinrichtung in der Podestplatte (4) im an die Serviceöffnung (8) anschließenden Podestplattenbereich angeordnet ist, insbesondere in einem der Serviceöffnung (8) unmittelbar zugeordneten und/oder benachbarten Randbereich, angeordnet ist.

15. Biogasanlagen-Fermenterbehälter nach einem der Ansprüche 10 bis 14, **dadurch gekennzeichnet, dass** die Podestplatte (4) in Verbindung mit einer im wesentlichen horizontalen und/oder aus einem festem Material, vorzugsweise aus Beton, hergestellten Behälterdeckenwand, in eine deckenwandseitige Ausnehmung eingesetzt und/oder aufgesetzt ist.

16. Biogasanlagen-Fermenterbehälter nach einem der Ansprüche 10 bis 14, **dadurch gekennzeichnet, dass** die Podestplatte (4) mittels einer Befestigungseinrichtung (6) gasdicht und fest an einer Behälterwand (2), insbesondere einer Behälterseitenwand, des Fermenterbehälters (1) angebunden ist, und
dass die Podestplatte (4) einen Foliendach-Anschlussbereich (12) aufweist, an dem das Foliendach (3), insbesondere ein Foliendach-Randbereich, mittelbar oder unmittelbar gasdicht angebunden ist.

17. Biogasanlagen-Fermenterbehälter nach Anspruch 16, **dadurch gekennzeichnet, dass** die Podestplatte (4) mittels einer Abstützeinrichtung (10), insbesondere mit einem Abstützgestell, von unten her abgestützt und am Fermenterbehälter-Innenraumwandbereich angebunden und festgelegt ist, wobei bevorzugt vorgesehen ist, dass die Gasschürze (16) so zwischen zwei beabstandeten Anschlussstützen der Abstützeinrichtung (10) angeordnet ist, dass die Anschlussstützen die rechteckförmig ausgebildete Gasschürze (16) auf gegenüberliegenden Rechtecklängsseiten der Gasschürze (16) abstützen.

18. Serviceeinrichtung für einen Biogasanlagen-Fermenterbehälter, bei der eine mittels wenigstens eines Deckels (15) verschließbare Serviceöffnung (8) in einer begehbaren Podestplatte (4) ausgebildet ist, von der eine die Serviceöffnung (8) ringförmig umschliessende Gasschürze (16) im montierten Zustand der Podestplatte (5) schachtartig nach unten in einen Fermenterbehälter-Innenraum (14) einragt, wobei eine Lagerstelle (18) eines Führungsmastes (17), an dem ein Tauchgerät (44), höhenverstellbar geführt ist, in der Podestplatte (4) im an die Serviceöffnung (8) anschließenden Podestplattenbereich ausgebildet und/oder angeordnet ist" wobei das Tauchgerät (44) mit einer außerhalb des Fermenterbehälters (1) angeordneten Tauchgerät-Höhenverstelleinrichtung (24) gekoppelt ist, die eine Antriebswelle (23) aufweist, die zusammen mit einer Lagerwelle (19) einer Führungsmast-Schwenkeinrichtung in einer Hohlwellenanordnung (25) durch die Lagerstelle (18) der Serviceeinrichtung hindurch in den Fermenterbehälter-Innenraum (14) geführt ist.

19. Serviceeinrichtung nach Anspruch 18, **dadurch gekennzeichnet, dass** an der Gasschürze (16) innenwandseitig, bevorzugt im oberen podestplattennahen und/oder deckelnahen Bereich, wenigstens ein verschließbarer Auslass, insbesondere wenigstens ein verschließbarer Gasauslass (61), angeordnet ist, der im geöffneten Zustand und bei in die Flüssigkeit eingetauchter Gasschürze (16) eine Strömungsverbindung zum Fermenterbehälter-Innenraum (14) herstellt, insbesondere zur Gasphase (60) im Fermenterbehälter-Innenraum (14) herstellt.

## Claims

1. Biogas plant fermenter tank comprising a service device which has a top-side service opening (8) which can be closed by means of a covering device (15) and through which a submersible unit (44) which is guided height-adjustably on a guide mast (17) is accessible for maintenance and service operations,
wherein the guide mast (17) is mounted in a bearing point (18) of the service device so as to be rotatable and/or pivotable by means of a bearing shaft (19), wherein, in order to rotate or pivot the guide mast (17), the bearing shaft (19) is coupled by means of a guide mast pivoting device (21) arranged outside the fermenter tank (1),
wherein the submersible unit (44) is coupled to a submersible unit height adjustment device (24) which is arranged outside the fermenter tank (1) and which has a drive shaft (23) which, together with the bearing shaft (19) of the guide mast pivoting device, is guided in a hollow shaft arrangement (25) through the bearing point (18) of the service device into the fermenter tank interior (14), and
wherein the drive shaft (23) interacts directly or indirectly in the fermenter tank interior (14) with an adjusting element (28) by means of which the submersible unit (44) is height-adjustable on the guide mast (17).

2. Biogas plant fermenter tank according to Claim 1, **characterized in that** the adjusting element is formed by a threaded spindle (28) extending parallel to the guide mast (17) and mounted rotatably there, wherein the threaded spindle (28) engages through a submersible unit-side spindle nut device (45) in such a way that, during a rotary actuation of the threaded spindle (28) by means of the drive shaft, the submersible unit (44), in dependence on the direction of rotation of the threaded spindle, is displaceable upwardly or downwardly along the guide mast (17).

3. Biogas plant fermenter tank according to Claim 1 or 2, **characterized in that** the hollow shaft arrangement (25) is formed in such a way that either the bearing shaft (19) of the guide mast pivoting device (21) or the drive shaft (23) of the submersible unit height adjustment device (24) is formed as a hollow shaft which is rotatably mounted, in particular by way of a plain bearing or ball bearing, on and/or in the bearing point (18) of the service device, and in which the respective other shaft is rotatably received and mounted, in particular by way of a plain bearing or ball bearing.

4. Biogas plant fermenter tank according to one of the preceding claims, **characterized in that** the drive shaft (23) of the submersible unit height adjustment device (24) has in the fermenter tank interior (14) a drive shaft drive element (26), in particular a drive gearwheel, which interacts directly or indirectly with an adjusting element drive element (27), in particular a threaded spindle drive element, preferably a threaded spindle gearwheel, coupled directly or indirectly to the adjusting element (28), in particular a threaded spindle.

5. Biogas plant fermenter tank according to Claims 3 and 4, **characterized in that** the bearing shaft (19) of the guide mast pivoting device (21) is formed as a hollow shaft through which the drive shaft (23) of the submersible unit height adjustment device (24) is guided into the fermenter tank interior (14), wherein the bearing shaft (19) is coupled to a bearing bridge (52) which is connected directly or indirectly to the guide mast (17) and which contactlessly bridges the drive shaft drive element (26) and/or receives it in a bridge-side clearance or receptacle, or **in that**, as an alternative, the drive shaft (23) of the submersible unit height adjustment device (24) is formed as a hollow shaft which has the drive shaft drive element (26) and through which the bearing shaft (19) of the guide mast pivoting device (21) is guided into the fermenter tank interior (14).

6. Biogas plant fermenter tank according to one of the preceding claims, **characterized in that** the guide mast pivoting device (21) is formed by a manually actuable pivoting lever (50) coupled to the bearing shaft (19) or by a drive motor (20), wherein an output shaft of the drive motor (20) forms the bearing shaft (19) of the guide mast pivoting device (21) or is a constituent part of the bearing shaft (19) of the guide mast pivoting device or is directly or indirectly coupled to the bearing shaft of the guide mast pivoting device (21) with a rotation-transmitting action.

7. Biogas plant fermenter tank according to one of the preceding claims, **characterized in that** the submersible unit height adjustment device (24) is formed by a manually actuable crank device (55) coupled to the drive shaft (23) or by a drive motor (24), wherein an output shaft of the drive motor (24) forms the drive shaft (23) of the submersible unit height adjustment device (24) or is a constituent part of the drive shaft (23) of the submersible unit height adjustment device (24) or is coupled directly or indirectly to the drive shaft (23) of the submersible unit height adjustment device (24) with a movement-transmitting action.

8. Biogas plant fermenter tank according to one of the preceding claims, **characterized in that** the hollow shaft arrangement (25) is formed in such a way that the hollow shaft is rotatably and/or pivotably mounted, in particular by way of a plain bearing or a ball bearing, in a sleeve (36) assigned to the bearing point (18) of the service device, wherein the sleeve (36) further has a fastening flange (37) by means of which the sleeve (36) is arranged around the bearing point (18) of the service device, in particular around the bearing point (18) of a platform panel (4) having the service opening (8), so as to be fixedly and non-rotatably connected to the service device, in particular to the platform panel (4).

9. Biogas plant fermenter tank according to one of the preceding claims, **characterized in that** a gas apron (16) which projects downwardly into the fermenter tank interior (14) in a shaft-like manner in the sealing position is arranged around the service opening (8) and, at least during maintenance and service operations, is submerged into the liquid substrate to be fermented which is received in the fermenter tank (1) and separates the gas phase region (59), which is annularly enclosed by the gas apron (16), below the openable service opening (8) and above the substrate (58) in a gas-tight manner from the remainder of the gas phase region (60) of the fermenter tank interior (14), wherein it is particularly provided that the gas apron (16) is formed by a rigid, non-displaceable and non-flexible tubular stub-like collar projecting permanently down from the service opening (8) in the direction of the fermenter tank interior (14).

10. Biogas plant fermenter tank according to one of the preceding claims, **characterized in that** the service device is or has a preferably walk-on platform panel (4) which is arranged on the top side and in which the service opening (8) is formed.

11. Biogas plant fermenter tank according to Claims 9 and 10, **characterized in that** the gas apron (16) annularly enclosing the service opening (8) projects downwardly from the platform panel (4).

12. Biogas plant fermenter tank according to Claim 11, **characterized in that** the gas apron (16) is formed by a separate component which is connected, in particular non-positively and/or in a material-bonded manner, to the platform panel (4), or **in that** the gas apron (16) is an integral constituent part of the platform panel (4) and is connected thereto by being made from one and the same material and/or connected thereto in one piece, in particular being formed by tabs bent out of the platform panel (4).

13. Biogas plant fermenter tank according to one of Claims 10 to 12, **characterized in that** the covering device which closes the service opening (8) in the platform panel (4), preferably in a gas-tight manner, is formed by at least one cover (15).

14. Biogas plant fermenter tank according to one of Claims 10 to 13, **characterized in that** the bearing point (18) of the service device in the platform panel (4) is arranged in the platform panel region adjoining the service opening (8), in particular in an edge region directly assigned and/or adjacent to the service opening (8).

15. Biogas plant fermenter tank according to one of Claims 10 to 14, **characterized in that** the platform panel (4), in conjunction with a tank top wall which is substantially horizontal and/or produced from a solid material, preferably from concrete, is inserted into and/or placed onto a top wall-side recess.

16. Biogas plant fermenter tank according to one of Claims 10 to 14, **characterized in that** the platform panel (4) is fixedly attached in a gas-tight manner to a container wall (2), in particular a container side wall, of the fermenter tank (1) by means of a fastening device (6), and
**in that** the platform panel (4) has a film roof connection region (12) to which the film roof (3), in particular a film roof edge region, is attached directly or indirectly in a gas-tight manner.

17. Biogas plant fermenter tank according to Claim 16, **characterized in that** the platform panel (4) is supported from below by means of a supporting device (10), in particular by a supporting framework, and attached and fastened to the fermenter tank interior wall region, wherein it is preferably provided that the gas apron (16) is arranged between two spaced-apart connection supports of the supporting device (10) in such a way that the connection supports support the rectangularly shaped gas apron (16) on opposite rectangle longitudinal sides of the gas apron (16).

18. Service device for a biogas plant fermenter tank, in which a service opening (8) which can be closed by means of at least one cover (15) is formed in a walk-on platform panel (4) from which, in the mounted state of the platform panel (5), a gas apron (16) annularly enclosing the service opening (8) projects downwardly in a shaft-like manner into a fermenter tank interior (14), wherein a bearing point (18) of a guide mast (17), on which a submersible unit (44) is guided height-adjustably, in the platform panel (4) is formed and/or arranged in the platform panel region adjoining the service opening (8),
wherein the submersible unit (44) is coupled to a submersible unit height adjustment device (24) which is arranged outside the fermenter tank (1) and which has a drive shaft (23) which, together with a bearing shaft (19) of a guide mast pivoting device, is guided in a hollow shaft arrangement (25) through the bearing point (18) of the service device into the fermenter tank interior (14).

19. Service device according to Claim 18, **characterized in that** at least one closable outlet, in particular at least one closable gas outlet (61), is arranged on the inner wall side of the gas apron (16), preferably in the upper region close to the platform panel and/or close to the cover, which outlet, in the opened state, and with the gas apron (16) submerged into the liquid, produces a flow connection to the fermenter tank interior (14), in particular to the gas phase (60) in the fermenter tank interior (14).

## Revendications

1. Récipient de fermentation pour installation de biogaz doté d'un dispositif de service comportant une ouverture de service (8) située du côté de plafond pouvant être refermée au moyen d'un dispositif de recouvrement (15) à travers lequel un appareil plongeur (44) guidé de façon réglable en hauteur pour les travaux d'entretien et de service est accessible au niveau d'un mât de guidage (17) ;
le mât de guidage (17) étant disposé de façon à pouvoir tourner et/ou pivoter à l'aide d'un arbre de palier (19) dans un palier (18) du dispositif de service, l'arbre de palier (19) étant couplé pour faire tourner ou pivoter le mât de guidage (17) au moyen d'un dispositif de pivotement de mât de guidage (21) disposé à l'extérieur du récipient de fermentation (1) ;
l'appareil plongeur (44) étant couplé à un dispositif de réglage en hauteur d'appareil plongeur (24) disposé à l'extérieur du récipient de fermentation (1) et comportant un arbre d'entraînement (23) guidé conjointement avec l'arbre de palier (19) du dispositif de pivotement de mât de guidage dans un agencement d'arbre creux (25) à travers le palier (18) du dispositif de service, dans l'espace intérieur de récipient de fermentation (14) ; et
l'arbre d'entraînement (23) interagissant directement ou indirectement avec un élément de réglage (28) dans l'espace intérieur de récipient de fermentation (14) à l'aide duquel l'appareil plongeur (44) peut être réglé en hauteur au niveau du mât de guidage (17).

2. Récipient de fermentation pour installation de biogaz selon la revendication 1, **caractérisé en ce que** l'élément de réglage est formé par une broche filetée (28) s'étendant parallèlement au mât de guidage (17) et est disposé à cet endroit de façon à pouvoir pivoter, la broche filetée (28) engrenant de telle sorte un dispositif à écrou de broche (45) du côté de l'appareil plongeur que l'appareil plongeur (44) puisse être déplacé vers le haut ou vers le bas par un actionnement en rotation de la broche filetée (28) à l'aide de l'arbre d'entraînement, en fonction de la direction de rotation de la broche filetée, le long du mât de guidage (17).

3. Récipient de fermentation pour installation de biogaz selon la revendication 1 ou 2, **caractérisé en ce que** l'agencement d'arbre creux (25) est réalisé de telle sorte que soit l'arbre de palier (19) du dispositif de pivotement de mât de guidage (21) soit l'arbre d'entraînement (23) du dispositif de réglage en hauteur d'appareil plongeur (24) prend la forme d'un arbre creux disposé de façon à tourner au niveau du palier (18) du dispositif de service et/ou dans celui-ci, notamment disposé de façon glissante ou à la façon d'une sphère et est logé et disposé de façon à pouvoir tourner dans l'autre arbre respectivement, notamment disposé de façon glissante ou à la façon d'une sphère.

4. Récipient de fermentation pour installation de biogaz selon l'une quelconque des revendications précédentes, **caractérisé en ce que** l'arbre d'entraînement (23) du dispositif de réglage en hauteur d'appareil plongeur (24) comporte dans l'espace intérieur de récipient de fermentation (14) un élément d'entraînement d'arbre d'entraînement (26), notamment une roue dentée d'entraînement, interagissant directement ou indirectement avec un élément d'entraînement d'élément de réglage (27) couplé directement ou indirectement à l'élément de réglage (28), notamment à une broche filetée, notamment avec un élément d'entraînement de broche filetée, de façon préférée une roue dentée de broche filetée.

5. Récipient de fermentation pour installation de biogaz selon la revendication 3 et 4, **caractérisé en ce que** l'arbre de palier (19) du dispositif de pivotement de mât de guidage (21) prend la forme d'un arbre creux à travers lequel l'arbre d'entraînement (23) du dispositif de réglage en hauteur d'appareil plongeur (24) est guidé dans l'espace intérieur de récipient de fermentation (14), l'arbre de palier (19) étant couplé à un pont de palier (52) relié directement ou indirectement au mât de guidage (17), ledit arbre chevauchant sans contact l'élément d'entraînement d'arbre d'entraînement (26) et/ou logeant dans un espace de réception ou un espace libre situé du côté du pont ou en variante, l'arbre d'entraînement (23) du dispositif de réglage en hauteur d'appareil plongeur (24) étant réalisé sous la forme d'un arbre creux comportant l'élément d'entraînement d'arbre d'entraînement (26) et à travers lequel l'arbre de palier (19) du dispositif de pivotement de mât de guidage (21) est disposé dans l'espace intérieur de récipient de fermentation (14).

6. Récipient de fermentation pour installation de biogaz selon l'une quelconque des revendications précédentes, **caractérisé en ce que** le dispositif de pivotement de mât de guidage (21) est formé par un levier pivotant (50) actionnable manuellement couplé à l'arbre de palier (19) ou par un moteur d'entraînement (20), un arbre d'entraînement en sortie du moteur d'entraînement (20) formant l'arbre de palier (19) du dispositif de pivotement de mât de guidage (21) ou faisant partie de l'arbre de palier (19) du dispositif de pivotement de mât de guidage ou étant couplé directement ou indirectement, avec transfert de rotation, à l'arbre de palier du dispositif de pivotement de mât de guidage (21).

7. Récipient de fermentation pour installation de biogaz selon l'une quelconque des revendications précédentes, **caractérisé en ce que** le dispositif de réglage en hauteur d'appareil plongeur (24) est formé par un dispositif de vilebrequin (55) actionnable manuellement couplé à l'arbre d'entraînement (23) ou par un moteur d'entraînement en entrée (34), un arbre d'entraînement en sortie du moteur d'entraînement (34) formant l'arbre d'entraînement (23) du dispositif de réglage en hauteur d'appareil plongeur (24) ou faisant partie de l'arbre d'entraînement (23) du dispositif de réglage en hauteur d'appareil plongeur (24) ou étant couplé directement ou indirectement, par transfert de mouvement, à l'arbre d'entraînement (23) du dispositif de réglage en hauteur d'appareil plongeur (24).

8. Récipient de fermentation pour installation de biogaz selon l'une quelconque des revendications précédentes, **caractérisé en ce que** l'agencement d'arbre creux (25) est réalisé de telle sorte que l'arbre creux est disposé de façon à pouvoir tourner et/ou pivoter dans une douille (36) associée au palier (18) du dispositif de service, notamment disposé de façon à pouvoir glisser ou à la façon d'une sphère, la douille (36) comportant en outre une bride de fixation (37) à l'aide de laquelle la douille (36) est reliée fixement et sans torsion au dispositif de service, notamment la plaque surélevée (4), autour du palier (18) du dispositif de service, notamment autour du palier (18) d'une plaque surélevée (4) comportant l'ouverture de service (8).

9. Récipient de fermentation pour installation de biogaz selon l'une quelconque des revendications précédentes, **caractérisé en ce qu'**un tablier de gaz (16) rentrant, à la façon d'un puits, dans la position étanche, est disposé vers le bas dans l'espace intérieur de récipient de fermentation (14), autour de l'ouverture de service (8), ledit tablier plongeant, au moins en cas de travaux d'entretien et de service, dans le substrat fluide à fermenter logeant dans le récipient de fermentation (1) et séparant la zone de phase de gaz (59), entourée de forme annulaire par le tablier de gaz (16) en-dessous de l'ouverture de service (8) ouvrable ainsi qu'au-dessus du substrat (58), de façon étanche aux gaz, du restant de la zone de phase de gaz (60) de l'espace intérieur de récipient de fermentation (14), sachant que l'on prévoit de façon préférée que le tablier de gaz (16) soit formé par un col rigide, non flexible et ne pouvant pas être déplacé ainsi que ressortant, à la façon d'une tubulure, hors de l'ouverture de service (8), de façon durable, en direction de l'espace intérieur de récipient de fermentation (14).

10. Récipient de fermentation pour installation de biogaz selon l'une quelconque des revendications précédentes, **caractérisé en ce que** le dispositif de service est ou comporte une plaque surélevée (4) disposée du côté de plafond, de préférence réalisée de façon accessible dans l'ouverture de service (8).

11. Récipient de fermentation pour installation de biogaz selon la revendication 9 et 10, **caractérisé en ce que** le tablier de gaz (16) entourant l'ouverture de service (8) en forme d'anneau ressort vers le bas hors de la plaque surélevée (4).

12. Récipient de fermentation pour installation de biogaz selon la revendication 11, **caractérisé en ce que** le tablier de gaz (16) est formé par un composant séparé relié à la plaque surélevée (4), notamment relié par complémentarité de forces et/ou de matières ou que le tablier de gaz (16) fait partie intégrante de la plaque surélevée (4) et est relié à celle-ci avec unité de matière et/ou d'un seul tenant, notamment formé par des brides incurvées hors de la plaque surélevée (4).

13. Récipient de fermentation pour installation de biogaz selon l'une quelconque des revendications 10 à 12, **caractérisé en ce que** le dispositif de recouvrement fermant l'ouverture de service (8) dans la plaque surélevée (4), la fermant de préférence de façon étanche aux gaz, est formé par au moins un cache (15).

14. Récipient de fermentation pour installation de biogaz selon l'une quelconque des revendications 10 à 13, **caractérisé en ce que** le palier (18) du dispositif de service est disposé dans la plaque surélevée (4), dans la zone de plaque surélevée, connexe à l'ouverture de service (8), notamment dans une zone de bordure connexe à l'ouverture de service (8) et/ou directement associée à elle.

15. Récipient de fermentation pour installation de biogaz selon l'une quelconque des revendications 10 à 14, **caractérisé en ce que** la plaque surélevée (4) est insérée et/ou placée dans un évidement situé du côté de paroi de plafond, en liaison avec une paroi de plafond de récipient pour l'essentiel horizontale et/ou fabriquée à partir d'un matériau solide, de préférence en béton.

16. Récipient de fermentation pour installation de biogaz selon l'une quelconque des revendications 10 à 14, **caractérisé en ce que** la plaque surélevée (4) est raccordée, de façon étanche aux gaz, au moyen d'un dispositif de fixation (6) et fixement à une paroi de récipient (2), notamment une paroi de récipient latérale, du récipient de fermentation (1) ; et que la plaque surélevée (4) comporte une zone de raccordement de toiture plate (12) à laquelle la toiture plate (3), notamment une zone de bordure de toiture plate, est raccordée directement ou indirectement de façon étanche aux gaz.

17. Récipient de fermentation pour installation de biogaz selon la revendication 16, **caractérisé en ce que** la plaque surélevée (4) est étançonnée par en bas à l'aide d'un dispositif d'étançonnage (10), notamment avec un châssis d'étançonnage et est raccordée et fixée à la zone de paroi d'espace intérieur de récipient de fermentation, sachant que l'on prévoit de façon préférée que le tablier de gaz (16) soit disposé de telle sorte, entre deux étançons de raccordement espacés du dispositif d'étançonnage (10), que les étançons de raccordement étançonnent le tablier de gaz (16) prenant une forme de rectangle sur les côtés longitudinaux opposés du rectangle du tablier de gaz (16).

18. Dispositif de service pour un récipient de fermentation pour installation de biogaz, dans lequel une ouverture de service (8) pouvant être refermée à l'aide d'au moins un cache (15) est réalisée dans une plaque surélevée (4) accessible à partir de laquelle un tablier de gaz (16) entourant de façon annulaire l'ouverture de service (8) rentrant vers le bas, à l'état monté de la plaque surélevée (5), à la façon d'un puits, dans un espace intérieur de récipient de fermentation (14), un palier (18) d'un mât de guidage (17) au niveau duquel un appareil plongeur (44) peut être réglé en hauteur étant réalisé et/ou disposé dans la plaque surélevée (4), dans la zone de plaque surélevée connexe à l'ouverture de service (8) ;
l'appareil plongeur (44) étant couplé à un dispositif de réglage en hauteur d'appareil plongeur (24) disposé à l'extérieur du récipient de fermentation (1) et comportant un arbre d'entraînement (23) guidé conjointement avec un arbre de palier (19) d'un dispositif de pivotement de mât de guidage dans un agencement d'arbre creux (25), à travers le palier (18) du dispositif de service, dans l'espace intérieur de récipient de fermentation (14).

19. Dispositif de service selon la revendication 18, **caractérisé en ce qu'**au moins une sortie pouvant être refermée, notamment au moins une sortie de gaz (61) pouvant être refermée, est disposée au niveau du tablier de gaz (16) du côté de la paroi intérieure, de façon préférée dans la zone située à proximité de la plaque surélevée supérieure et/ou à proximité du cache établissant, à l'état ouvert en en cas de tablier de gaz (16) plongeant dans le fluide, une liaison d'écoulement par rapport à l'espace intérieur de récipient de fermentation (14), notamment par rapport à la phase de gaz (60), dans l'espace intérieur de récipient de fermentation (14).
